# EUROPEAN PATENT SPECIFICATION

(11) **EP 0 730 648 B1**
(45) Date of publication and mention of the grant of the patent: **11.08.2004**
(21) Application number: 95904201.1
(22) Date of filing: 02.12.1994
(51) Int. Cl.: C12N 15/12, C12Q 1/68, A01K 67/00, A61K 48/00, C07K 14/47

(54) **HUMAN MUTATOR GENE hMSH2 AND HEREDITARY NON POLYPOSIS COLORECTAL CANCER**
MENSCHLICHES MUTATOR-GEN NMSH2 UND SEINE VERBINDUNG ZUM HEREDITAREN, NICHTPOLYPÖSEN KOLORREKTALEN KARZINOM
GENE MUTATEUR HUMAIN hMSH2 ET CANCER COLO-RECTAL NON POLYPOSIQUE HEREDITAIRE

(30) Priority: 02.12.1993 US 160295
(43) Date of publication of application: 11.09.1996
(73) Proprietor: THE JOHNS HOPKINS UNIVERSITY, Baltimore, MD 21205-2109 (US); DE LA CHAPELLE, Albert, 00140 Helsingfors (FI)
(72) Inventor: DE LA CHAPELLE, Albert, FIN-00140 Helsingfors (FI); VOGELSTEIN, Bert, Baltimore, MD 21208 (US); KINZLER, Kenneth, W., Baltimore, MD 21234 (US)
(74) Representative: Tombling, Adrian George
(86) International application number: PCT/US1994/013805
(87) International publication number: WO 1995/015381

(56) References cited:
- SCIENCE, vol.260, 7 May 1993 pages 810 - 812 PELTOMAEKI, P. ET AL. 'Genetic mapping of a locus predisposing to human colorectal cancer' cited in the application
- NATURE, vol.363, 10 June 1993 pages 558 - 561 IONOV, Y. ET AL. 'Ubiquitous somatic mutations in simple repeated sequences reveal a new mechanism for colonic carcinogenesis' cited in the application
- NATURE, vol.365, 16 September 1993 pages 274 - 276 STRAND, M. ET AL. 'Destabilization of tracts of simple repetitive DNA in yeast by mutations affecting DNA mismatch repair' cited in the application
- CELL, vol.75, 3 December 1993 pages 1027 - 1038 FISHEL, R. ET AL. 'The human mutator gene homolog MSH2 and its association with hereditary nonpolyposis colon cancer'
- CELL, vol.75, 17 December 1993 pages 1215 - 1225 LEACH, F.S. ET AL. 'Mutations of a mutS homolog in hereditary nonpolyposis colorectal cancer'
- CELL, vol.75, 17 December 1993 pages 1227 - 1236 PARSONS, R. ET AL. 'Hypermutability and mismatch repair deficiency in RER+ tumor cells'

### SEQUENCE LISTING

(1) GENERAL INFORMATION:
   (i) APPLICANT: Vogelstein, Bert Kinzler, Kenneth W. de la Chappelle, Albert
   (ii) TITLE OF INVENTION: Mutator Gene and Hereditary Non-Polyposis Colorectal Cancer
   (iii) NUMBER OF SEQUENCES: 16
   (iv) CORRESPONDENCE ADDRESS:
      (A) ADDRESSEE: Banner, Birch, McKie, and Beckett
      (B) STREET: 1001 G Street, N.W.
      (C) CITY: Washington
      (D) STATE: D.C.
      (E) COUNTRY: USA
      (F) ZIP: 20001
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US 08/160295
      (B) FILING DATE: 02-DEC-1993
      (C) CLASSIFICATION:
   (viii) ATTORNEY/AGENT INFORMATION:
      (A) NAME: Kagan, Sarah A.
      (B) REGISTRATION NUMBER: 32,141
      (C) REFERENCE/DOCKET NUMBER: 01107.44900
   (ix) TELECOMMUNICATION INFORMATION:
      (A) TELEPHONE: 202.508.9100
      (B) TELEFAX: 202.508.9299
      (C) TELEX: 197430 BBMB UT
(2) INFORMATION FOR SEQ ID NO:1:
   (i) SEQUENCE CHARACTERISTICS:
      (A) LENGTH: 2947 base pairs
      (B) TYPE: nucleic acid
      (C) STRANDEDNESS: double
      (D) TOPOLOGY: linear
   (ii) MOLECULE TYPE: CDNA
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:1:
   GGCGGGAAAC AGCTTAGTGG GTGTGGGGTC GCGCATTTTC TTCAACCAGG AGGTGAGGAG 60
   GTTTCGACAT GGCGGTGCAG CCGAAGGAGA CGCTGCAGTT GGAGAGCGCG GCCGAGGTCG 120
   GCTTCGTGCG CTTCTTTCAG GGCATGCCGG AGAAGCCGAC CACCACAGTG CGCCTTTTCG 180
   ACCGGGGCGA CTTCTATACG GCGCACGGCG AGGACGCGCT GCTGGCCGCC CGGGAGGTGT 240
   TCAAGACCCA GGGGGTGATC AAGTACATGG GGCCGGCAGG AGCAAAGAAT CTGCAGAGTG 300
   TTGTGCTTAG TAAAATGAAT TTTGAATCTT TTGTAAAAGA TCTTCTTCTG GTTCGTCAGT 360
   ATAGAGTTGA AGTTTATAAG AATAGAGCTG GAAATAAGGC ATCCAAGGAG AATGATTGGT 420
   ATTTGGCATA TAAGGCTTCT CCTGGCAATC TCTCTCAGTT TGAAGACATT CTCTTTGGTA 480
   ACAATGATAT GTCAGCTTCC ATTGGTGTTG TGGGTGTTAA AATGTCCGCA GTTGATGGCC 540
   AGAGACAGGT TGGAGTTGGG TATGTGGATT CCATACAGAG GAAACTAGGA CTGTGTGAAT 600
   TCCCTGATAA TGATCAGTTC TCCAATCTTG AGGCTCTCCT CATCCAGATT GGACCAAAGG 660
   AATGTGTTTT ACCCGGAGGA GAGACTGCTG GAGACATGGG GAAACTGAGA CAGATAATTC 720
   AAAGAGGAGG AATTCTGATC ACAGAAAGAA AAAAAGCTGA CTTTTCCACA AAAGACATTT 780
   ATCAGGACCT CAACCGGTTG TTGAAAGGCA AAAAGGGAGA GCAGATGAAT AGTGCTGTAT 840
   TGCCAGAAAT GGAGAATCAG GTTGCAGTTT CATCACTGTC TGCGGTAATC AAGTTTTTAG 900
   AACTCTTATC AGATGATTCC AACTTTGGAC AGTTTGAACT GACTACTTTT GACTTCAGCC 960
   AGTATATGAA ATTGGATATT GCAGCAGTCA GAGCCCTTAA CCTTTTTCAG GGTTCTGTTG 1020
   AAGATACCAC TGGCTCTCAG TCTCTGGCTG CCTTGCTGAA TAAGTGTAAA ACCCCTCAAG 1080
   GACAAAGACT TGTTAACCAG TGGATTAAGC AGCCTCTCAT GGATAAGAAC AGAATAGAGG 1140
   AGAGATTGAA TTTAGTGGAA GCTTTTGTAG AAGATGCAGA ATTGAGGCAG ACTTTACAAG 1200
   AAGATTTACT TCGTCGATTC CCAGATCTTA ACCGACTTGC CAAGAAGTTT CAAAGACAAG 1260
   CAGCAAACTT ACAAGATTGT TACCGACTCT ATCAGGGTAT AAATCAACTA CCTAATGTTA 1320
   TACAGGCTCT GGAAAAACAT GAAGGAAAAC ACCAGAAATT ATTGTTGGCA GTTTTTGTGA 1380
   CTCCTCTTAC TGATCTTCGT TCTGACTTCT CCAAGTTTCA GGAAATGATA GAAACAACTT 1440
   TAGATATGGA TCAGGTGGAA AACCATGAAT TCCTTGTAAA ACCTTCATTT GATCCTAATC 1500
   TCAGTGAATT AAGAGAAATA ATGAATGACT TGGAAAAGAA GATGCAGTCA ACATTAATAA 1560
   GTGCAGCCAG AGATCTTGGC TTGGACCCTG GCAAACAGAT TAAACTGGAT TCCAGTGCAC 1620
   AGTTTGGATA TTACTTTCGT GTAACCTGTA AGGAAGAAAA AGTCCTTCGT AACAATAAAA 1680
   ACTTTAGTAC TGTAGATATC CAGAAGAATG GTGTTAAATT TACCAACAGC AAATTGACTT 1740
   CTTTAAATGA AGAGTATACC AAAAATAAAA CAGAATATGA AGAAGCCCAG GATGCCATTG 1800
   TTAAAGAAAT TGTCAATATT TCTTCAGGCT ATGTAGAACC AATGCAGACA CTCAATGATG   1860
   TGTTAGCTCA GCTAGATGCT GTTGTCAGCT TTGCTCACGT GTCAAATGGA GCACCTGTTC   1920
   CATATGTACG ACCAGCCATT TTGGAGAAAG GACAAGGAAG AATTATATTA AAAGCATCCA   1980
   GGCATGCTTG TGTTGAAGTT CAAGATGAAA TTGCATTTAT TCCTAATGAC GTATACTTTG   2040
   AAAAAGATAA ACAGATGTTC CACATCATTA CTGGCCCCAA TATGGGAGGT AAATCAACAT   2100
   ATATTCGACA AACTGGGGTG ATAGTACTCA TGGCCCAAAT TGGGTGTTTT GTGCCATGTG   2160
   AGTCAGCAGA AGTGTCCATT GTGGACTGCA TCTTAGCCCG AGTAGGGGCT GGTGACAGTC   2220
   AATTGAAAGG AGTCTCCACG TTCATGGCTG AAATGTTGGA AACTGCTTCT ATCCTCAGGT   2280
   CTGCAACCAA AGATTCATTA ATAATCATAG ATGAATTGGG AAGAGGAACT TCTACCTACG   2340
   ATGGATTTGG GTTAGCATGG GCTATATCAG AATACATTGC AACAAAGATT GGTGCTTTTT   2400
   GCATGTTTGC AACCCATTTT CATGAACTTA CTGCCTTGGC CAATCAGATA CCAACTGTTA   2460
   ATAATCTACA TGTCACAGCA CTCACCACTG AAGAGACCTT AACTATGCTT TATCAGGTGA   2520
   AGAAAGGTGT CTGTGATCAA AGTTTTGGGA TTCATGTTGC AGAGCTTGCT AATTTCCCTA   2580
   AGCATGTAAT AGAGTGTGCT AAACAGAAAG CCCTGGAACT TGAGGAGTTT CAGTATATTG   2640
   GAGAATCGCA AGGATATGAT ATCATGGAAC CAGCAGCAAA GAAGTGCTAT CTGGAAAGAG   2700
   AGCAAGGTGA AAAAATTATT CAGGAGTTCC TGTCCAAGGT GAAACAAATG CCCTTTACTG   2760
   AAATGTCAGA AGAAAACATC ACAATAAAGT TAAAACAGCT AAAAGCTGAA GTAATAGCAA   2820
   AGAATAATAG CTTTGTAAAT GAAATCATTT CACGAATAAA AGTTACTACG TGAAAAATCC   2880
   CAGTAATGGA ATGAAGGTAA TATTGATAAG CTATTGTCTG TAATAGTTTT ATATTGTTTT   2940
   ATATTAA                                                             2947
(2) INFORMATION FOR SEQ ID NO:2:
   (i) SEQUENCE CHARACTERISTICS:
      (A) LENGTH: 934 amino acids
      (B) TYPE: amino acid
      (C) STRANDEDNESS: single
      (D) TOPOLOGY: linear
   (ii) MOLECULE TYPE: protein
   (iii) HYPOTHETICAL: YES
   (iv) ANTI-SENSE: NO
   (vi) ORIGINAL SOURCE:
      (A) ORGANISM: Homo sapiens
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:3:
   CTGGATCCAC NGGNCCNAAY ATG 23
(2) INFORMATION FOR SEQ ID NO:4:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:4:
   CTGGATCCRT ARTGNGTNRC RAA 23
(2) INFORMATION FOR SEQ ID NO:5:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:5:
   CCACAATGGA CACTTCTGC 19
(2) INFORMATION FOR SEQ ID NO:6:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (xi) SEQUENCE DESCRIPTION: SEQ ID NO:6:
   CACCTGTTCC ATATGTACG 19
(2) INFORMATION FOR SEQ ID NO:7:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:7:
   AAAATGGGTT GCAAACATGC 20
(2) INFORMATION FOR SEQ ID NO:8:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:8:
   GTGATAGTAC TCATGGCCC 19
(2) INFORMATION FOR SEQ ID NO:9:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: YES
   (xi) SEQUENCE DESCRIPTION: SEQ ID NO:9:
   AGATCTTCTT CTGGTTCGTC 20
(2) INFORMATION FOR SEQ ID NO:10:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:10:
   GCCAACAATA ATTTCTGGTG 20
(2) INFORMATION FOR SEQ ID NO:11:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:11:
   TGGATAAGAA CAGAATAGAG G 21
(2) INFORMATION FOR SEQ ID NO:12:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:12:
   CCACAATGGA CACTTCTGC 19
(2) INFORMATION FOR SEQ ID NO:13:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:13:
   CACCTGTTCC ATATGTACG 19
(2) INFORMATION FOR SEQ ID NO:14:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:14:
   AAAATGGGTT GCAAACATGC 20
(2) INFORMATION FOR SEQ ID NO:15:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:15:
   GTGATAGTAC TCATGGCCC 19
(2) INFORMATION FOR SEQ ID NO:16:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:16:
   GACAATAGCT TATCAATATT ACC 23

## Description

This invention was made using U.S. government grants from the NIH CA47527, CA09320, GM26449, CA09243, CA41183, CA42705 CA57435, and CA35494, as well as grants from the Department of Energy DOE/ERN/F139 and DE-FG 09291ER-61139. Therefore the U.S. government retains certain rights to the invention.

This application is a continuation-in-part of Serial No. 08/056,546, filed May 5, 1993.

### TECHNICAL FIELD OF THE INVENTION

The invention relates to a gene which predisposes individuals to colorectal and other cancers. In addition, it also relates to biochemical tests which can be used to identify drugs for treatment of affected individuals.

### BACKGROUND OF THE INVENTION

HNPCC (Lynch syndrome) is one of the most common cancer predisposition syndromes, affecting as many as 1 in 200 individuals in the western world (Lynch et al., 1993). Affected individuals develop tumors of the colon, endometrium, ovary and other organs, often before 50 years of age. Although the familial nature of this syndrome was discovered nearly a century ago (Warthin et al., 1913), the role of heredity in its causation remained difficult to define (Lynch et al., 1966). Recently, however, linkage analysis in two large kindreds demonstrated association with polymorphic markers on chromosome 2 (Peltomaki et al., 1993a). Studies in other families suggested that neoplasia in a major fraction of HNPCC kindreds is linked to this same chromosome 2p locus (Aaltonen et al., 1993).

HNPCC is defined clinically by the occurrence of early-onset colon and other specific cancers in first degree relatives spanning at least two generations (Lynch et al., 1993). The predisposition is inherited in an autosomal dominant fashion. It was initially expected that the gene(s) responsible for HNPCC was a tumor suppressor gene, as other previously characterized cancer predisposition syndromes with this mode of inheritance are caused by suppressor gene mutations (reviewed in Knudson, 1993). But the analysis of tumors from HNPCC patients suggested a different mechanism. Most loci encoding tumor suppressor genes undergo somatic losses during tumorigenesis (Stanbridge, 1990). In contrast, both alleles of chromosome 2p loci were found to be retained in HNPCC tumors (Aaltonen et al., 1993). During this search for chromosome 2 losses, however, it was noted that HNPCC tumors exhibited somatic alterations of numerous microsatellite sequences.

Widespread, subtle alterations of the cancer cell genome were first detected in a subset of sporadic colorectal tumors using the arbitrarily-primed polymerase chain reaction (Peinado et al., 1992). These alterations were subsequently found to represent deletions of up to 4 nucleotides in genomic polyA tracts (Ionov et al., 1993). Other studies showed that a similar, distinctive subgroup of sporadic tumors had insertions or deletions in a variety of simple repeated sequences, particularly microsatellite sequences consisting of dinucleotide or trinucleotide repeats (Ionov et al., 1993; Thibodeau et al., 1993; Aaltonen et al., 1993). Interestingly, these sporadic tumors had certain features in common with those developing in HNPCC kindreds, such as a tendency to be located on the right side of the colon and to be near-diploid. These and other data suggested that HNPCC and a subset of sporadic tumors were associated with a heritable defect causing replication errors (RER) of microsatellites (Ionov et al., 1993; Aaltonen et al., 1993).

The mechanism underlying the postulated defect could not be determined from the study of tumor DNA, but studies in simpler organisms provided an intriguing possibility (Levinson and Gutman, 1987; Strand et al., 1993). This work showed that bacteria and yeast containing defective mismatch repair genes manifest instability of dinucleotide repeats. The disruption of genes primarily involved in DNA replication or recombination had no apparent effect on the fidelity of microsatellite replication (reviewed in Kunkel, 1993). These pivotal studies suggested that defective mismatch repair might be responsible for the microsatellite alterations in the tumors from HNPCC patients (Strand et al., 1993).

WO 95/14085 discloses an incorrect sequence of *hMSH2.* Furthermore, the incorrect *hMSH2* sequence disclosed is not entitled to the earliest priority date and is not prior art to the present application.

WO 95/14085 discloses 3 fragments of the *hMSH2* sequence given in SEQ ID NO. 1 herein. The 3 framents are not encompassed by the present invention.

Thus there is a need in the art to identify the actual gene and protein responsible for hereditary non-polyposis colorectal cancer and the replication error phenotype found in both hereditary and sporadic tumors. Identification of the gene and protein would allow more widespread diagnostic screening for hereditary non-polyposis colorectal cancer than is currently possible. Identification of the involved gene and protein would also enable the rational screening of compounds for use in drug therapy of hereditary non-polyposis colorectal cancer, and would enable gene therapy for affected individuals.

### SUMMARY OF THE INVENTION

It is an object of the invention to provide a DNA molecule which when mutated is the genetic determinant for hereditary non-polyposis colorectal cancer.

It is another object of the invention to provide DNA molecules which contain specific mutations which cause hereditary non-polyposis colorectal cancer.

It is yet another object of the invention to provide methods of treating persons who are predisposed to hereditary non-polyposis colorectal cancer.

It is still another object of the invention to provide methods for determining a predisposition to cancer.

It is a further object of the invention to provide methods for screening test compounds to identify therapeutic agents for treating persons predisposed to hereditary non-polyposis colorectal cancer.

It is still another object of the invention to provide a protein which is important for human DNA mismatch repair.

It is yet another object of the invention to provide a transgenic animal for studying potential therapies for hereditary non-polyposis colorectal cancer.

These and other objects of the invention are provided by one or more of the embodiments described below. In one embodiment of the invention an isolated and purified DNA molecule is provided. The molecule has an *hMSH2* sequence of at least about 20 nucleotides of *hMSH2,* as shown in SEQ ID NO:1, wherein the *hMSH2* sequence does not consist of nucleotides 2085 to 2405, 2118 to 2135 or 2279 to 2296 of SEQ ID NO. 1.

In another embodiment of the invention an isolated and purified DNA molecule is provided. The DNA molecule has a sequence of at least about 20 nucleotides of an *hMSH2* allele found in a tumor wherein said DNA molecule contains a mutation relative to *hMSH2* shown in SEQ ID NO:1.

In yet another embodiment of the invention a method of treating a person predisposed to hereditary non-polyposis colorectal cancer is provided. The method prevents accumulation of somatic mutations. The method involves administering a DNA molecule which has a sequence of at least about 20 nucleotides of *hMSH2,* as shown in SEQ ID NO:1, to a person having a mutation in an *hMSH2* allele which predisposes the person to hereditary non-polyposis colorectal cancer, wherein said DNA molecule is sufficient to remedy the mutation in an *hMSH2* allele of the person.

In another embodiment of the invention a method is provided for determining a predisposition to cancer. The method involves testing a body sample of a human to ascertain the presence of a mutation in *hMSH2* which affects *hMSH2* expression or *hMSH2* protein function, the presence of such a mutation indicating a predisposition to cancer.

In still another embodiment of the invention a method is provided for screening to identify therapeutic agents which can prevent or ameliorate tumors. The screening method involves contacting a test compound with a purified *hMSH2* protein or a cell; determining the ability of the *hMSH2* protein or the cell to perform DNA mismatch repair, wherein a test compound which increases the ability of said *hMSH2* protein or said cell to perform DNA mismatch repair being a potential therapeutic agent.

In another embodiment of the invention an isolated and purified protein is provided. The protein has the sequence shown in SEQ ID NO:2.

In still another embodiment of the invention a transgenic animal is provided. The transgenic (nonhuman) animal maintains an *hMSH2* allele in its germline. The *hMSH2* allele is one which is found in humans having hereditary non-polyposis colorectal cancer or in RER⁺ tumors. Also provided are animals which have no wild-type *MSH2* alleles, due to mutations introduced.

Thus the present invention provides the art with the sequence of the gene responsible for hereditary non-polyposis colorectal cancer and information regarding the mechanism by which it causes tumors. This enables the art to practice a variety of techniques to identify persons at risk of developing a variety of cancers and to treat them to prevent such cancers from actually developing.

### BRIEF DESCRIPTION OF THE DRAWINGS

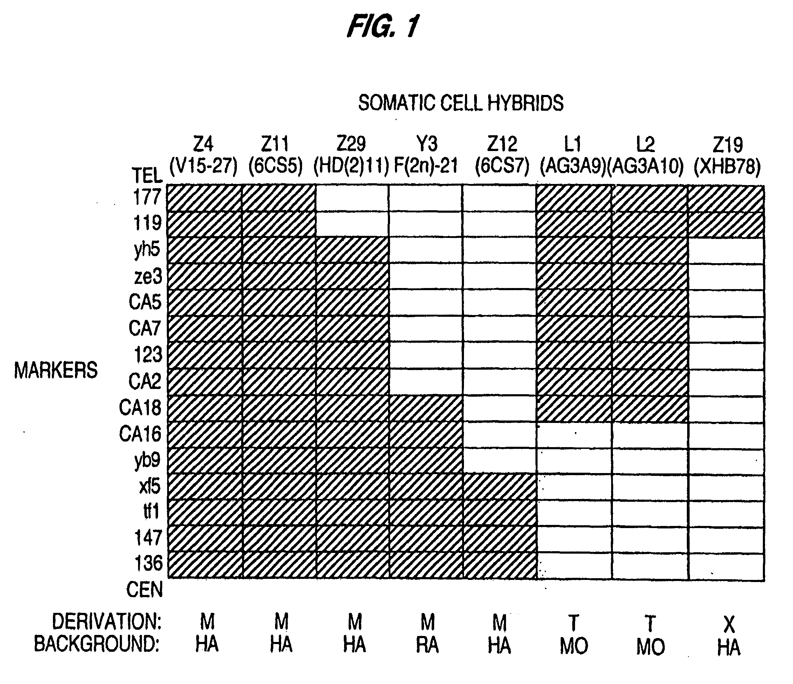

Figure 1 summarizes the markers retained in somatic cell hybrids used in locating the *hMSH2* gene.

PCR was used to determine whether each of the listed markers was present (black box) or absent (white box)in the indicated hybrid. The laboratory name of each hybrid and the formal name (in parentheses) is listed. The hybrid panel was also validated with ten additional polymorphic markers outside of the 136-177 region. M: hybrid derived from microcell-mediated chromosome 2 transfer; T: derived from t(X;2) translocation; X: derived from X-irradiated chromosome 2 donor. MO: mouse-human hybrid; HA: hamster-human hybrid; RA: rat-human hybrid; TEL: telomere; CEN: centromere.

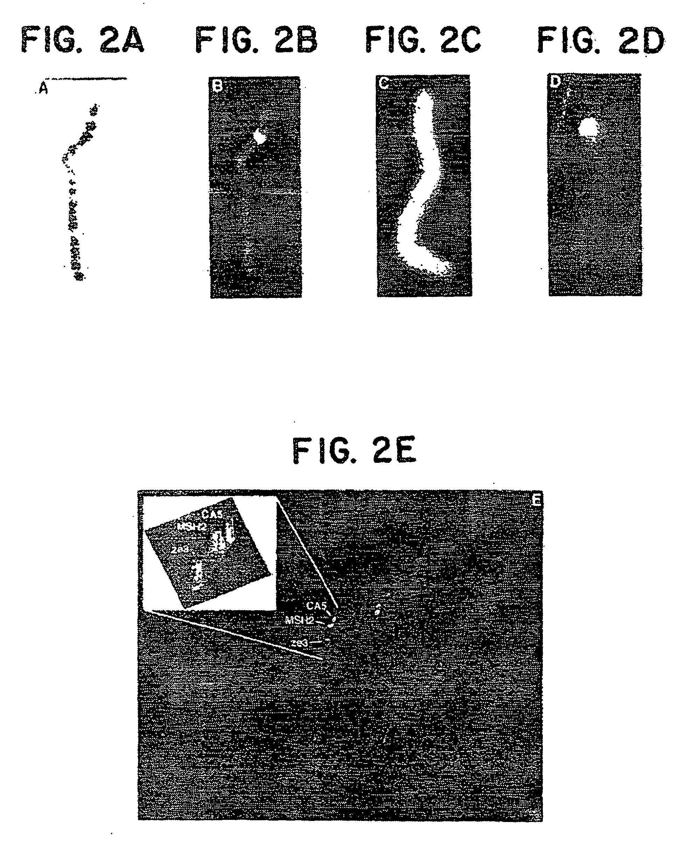

Figure 2 shows a FISH analysis which was used to determine the proximity and ordering of DNA sequences within chromosome band 2p16.

Panels 2A and 2B show FISH mapping of the 123 marker. Panel 2A shows G-banded metaphase chromosome 2. Panel 2B shows identical chromosome as in Panel 2A following FISH with a biotin-labeled P1 clone for the 123 marker. Results localize the 123 marker to chromosome band 2p16.3. Panels 2C and 2D show co-hybridization documenting the coincident localization of a microdissection (Micro-FISH) probe from chromosome 2p16 and the 123 marker. Panel 2C shows DAPI stained metaphase chromosome 2. Panel 2D shows simultaneous hybridization of the biotin-labeled 123 probe (appearing as an intensely staining smaller circle) and the Spectrum-Orange labeled 2p16 Micro-FISH probe (appearing as a diffusely staining larger circle). Panel 2E shows a representative example of an interphase nucleus simultaneously hybridized with P1 clones for CA5, *hMSH2* and ze3. The results were used to directly measure the distances between markers in order to establish the order and relative distance between markers (after Trask et al., 1989). Inset: The image processing program NIH *Image* was used to provide an average gray value displayed as a surface plot to support the length measurements and to graphically illustrate the relative order information. The surface plot presented defines the specified interphase chromosome and the relative order CA-*MSH2*-ze3.

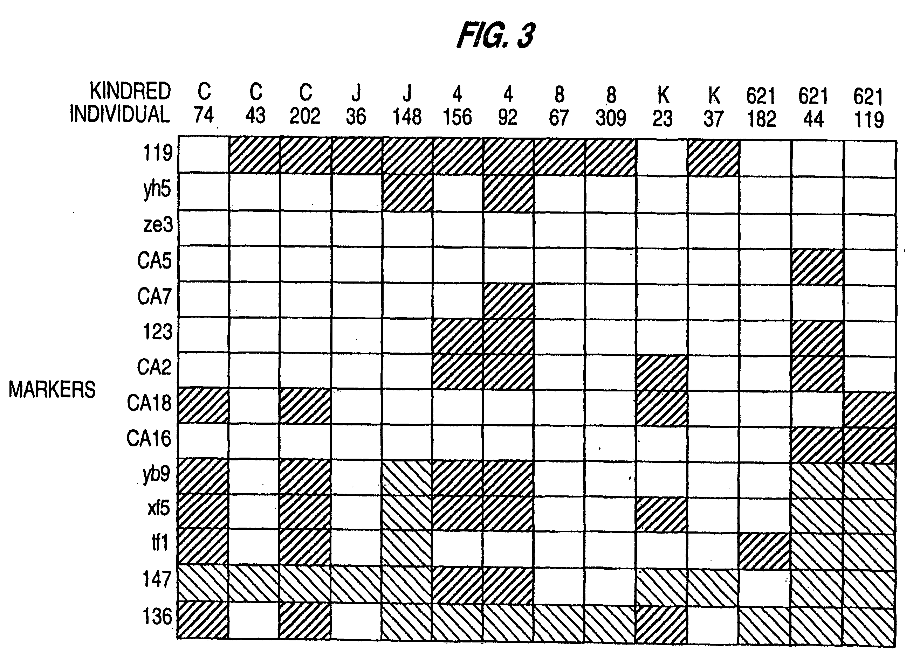

Figure 3 shows linkage analysis of HNPCC pedigrees.

All affected individuals in which meiotic recombination occurred between markers 119 and 136 are included. A black box indicates that the individual did not contain the allele associated with disease in his/her family or that the individual inherited an allele not associated with disease from his/her affected parent. A white box indicates that the individual had an allele which was the same size as the disease-associated allele. A hatched circle indicates that the marker was not studied. All individuals had colon or endometrial cancer at less than 55 years of age, or had progeny with such disease but did not indicate that the patient necessarily had disease-associated alleles because phase could usually not be determined.

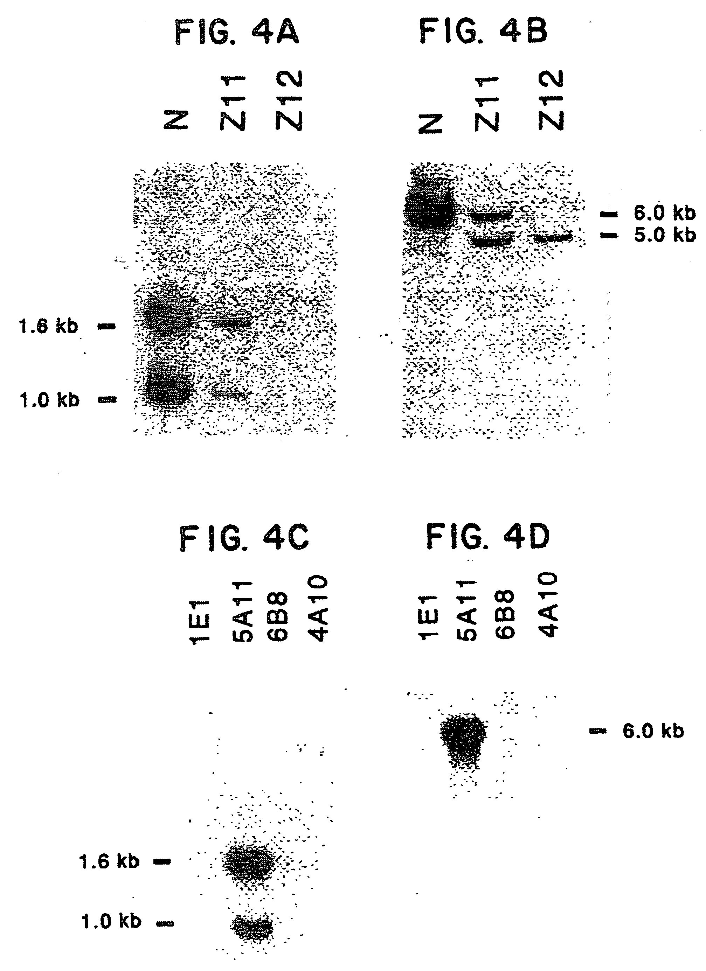

Figure 4 shows *hMSH2* gene localization.

Southern blots containing EcoRI (figures 4A and 4C) and PstI (figures 4B and 4D) digested DNA from the indicated somatic cell hybrids (figures 4A and 4B) or YAC clones (figures 4C and 4D) were hybridized with a radiolabelled insert from cDNA clone pNP-23. Southern blotting and hybridization were performed as described (Vogelstein et al., 1987). Autoradiographs are shown. The 5.0 kb PstI fragment in hybrids Z11 and Z12 is derived from hamster DNA.

Figure 5 shows the cDNA sequence of *hMSB2.*

An open reading frame (ORF) begins at nucleotide 1 and ends at nt 2802. The predicted amino acid sequence is shown. The sequence downstream of nt 2879 was not determined.

Figure 6 shows homology between yeast and human *MSH2* genes.

The predicted amino acid sequences of yeast (y) *MSH2* (Reenan and Kolodner, 1992) and human *MSH2* genes are compared within the region of highest homology. Blocks of similar amino acids are shaded.

Figure 7 shows germline and somatic mutations of *hMSH2.*

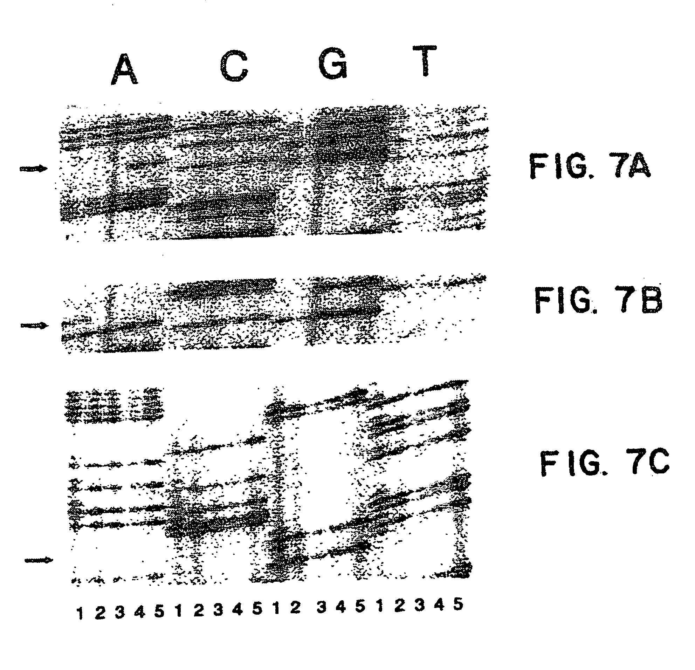

Autoradiographs of polyacrylamide gels containing the sequencing reactions derived from PCR products are shown. The 1.4 kb PCR products containing a conserved region of *hMSH2* were generated from genomic DNA samples as described in the Examples. Antisense primers were used in the sequencing reactions. The ddA mixes from each sequencing reaction were loaded in adjacent lanes to facilitate comparison, as were those for C, G, and T. The DNA samples were derived from the tumor (lane 1) and normal colon (lane 2) of patient Cx10, an RER- colon tumor cell line (lane 3), and lymphocytes of patients J-42 (lane 4) and J-143 (lane 5). Figure 7A: A transition (C to T at codon 622) in lymphocyte DNA can be observed in HNPCC patients J-42 and J-143. Figure 7B: A transition (C to T at nt codon 639) in tumor (lane 1) and normal colonic mucosa (lane 2) of patient Cx10. Figure 7C: A substitution of a TG dinucleotide for an A at codon 663 can be observed in DNA of the tumor of patient Cx10, (lane 1), but not in DNA from her normal colon (lane 2). Arrows mark the substitutions in panel A and B and the TG dinucleotide insertion site in panel C.

### DETAILED DESCRIPTION OF THE PREFERRED EMBODIMENTS

The disclosure of Serial No. 08/056,546, filed May 5, 1993, is expressly incorporated herein.

It is a discovery of the present invention that the gene responsible for hereditary non-polyposis colorectal cancer is *hMSH2,* a human analog of bacterial *MutS.* The cDNA sequence of *hMSH2* is shown in SEQ ID NO:1. This gene encodes a DNA mismatch repair enzyme. Mutation of the gene causes cells to accumulate mutations. For example, the observed replication error phenotype (RER⁺) found in both sporadic and hereditary non-polyposis colorectal cancer consists of variations (insertions and deletions) in microsatellite DNA. In yeast and bacteria defective *MutS*-related genes cause other types of mutations as well.

Useful DNA molecules according to the invention are those which will specifically hybridize to *hMSH2* sequences. Typically these are at least about 20 nucleotides in length and have the nucleotide sequence as shown in SEQ ID NO:1. Such molecules can be labeled, according to any technique known in the art, such as with radiolabels, fluorescent labels, enzymatic labels, sequence tags, etc. According to another aspect of the invention, the DNA molecules contain a mutation which has been found in tumors of HNPCC patients or in sporadic RER⁺ tumors. Such molecules can be used as allele-specific oligonucleotide probes to track a particular mutation through a family.

According to some aspects of the invention, it is desirable that the DNA encode all or a part of the *hMSH2* protein as shown in SEQ ID NO:2. To obtain expression of the protein the DNA sequence can be operably linked to appropriate control sequences, such as promotor, Kozak consensus, and terminator sequences.

A person who is predisposed to develop cancers due to inheritance of a mutant *hMSH2* allele can be treated by administration of a DNA molecule which contains all or a part of the normal *hMSH2* gene sequence as shown in SEQ ID NO:1. A portion of the gene sequence will be useful when it spans the location of the mutation which is present in the mutant allele, so that a double recombination event between the mutant allele and the normal portion "corrects" the defect present in the person. A portion of the gene can also be usefully administered when it encodes enough of the protein to express a functional DNA mismatch repair enzyme. Such a portion need not necessarily recombine with the mutant allele, but can be maintained at a separate locus in the genome or on an independently replicating vector. Means for administering DNA to humans are known in the art, and any can be used as is convenient. A variety of vectors are also known for this purpose. According to some techniques vectors are not required. Such techniques are well known to those of skill in the art.

Also contemplated as part of the present invention is the use of a combined anti-neoplastic therapy regimen. Such a combined regimen is useful for patients having an RER⁺ tumor, whether sporadic or associated with HNPCC. The regimen combines any standard anti-neoplastic therapy to which a patient can become resistant and *hMSH2* gene therapy, as described above. By remedying the defect present in RER⁺ cells, *i.e.,* an *hMSH2* mutation, the likelihood of the tumor developing a resistance mutation is greatly diminished. By delaying or preventing the onset of resistance, the life of cancer patients can be prolonged. In addition, such prevention of resistance allows a greater degree of tumor destruction by the therapeutic agent. Examples of anti-neoplastic therapies which can be combined with *hMSH2* gene therapy are hormones, radiation, cytotoxic drugs, cytotoxins, and antibodies.

Body samples can be tested to determine whether the *hMSH2* gene is normal or mutant. Mutations are those deviations from the sequence shown in SEQ ID NO:1 which are associated with disease and which cause a change in *hMSH2* protein function or expression. Such mutations include nonconservative amino acid substitutions, deletions, premature terminations and frameshifts. See Table I. Suitable body samples for testing include those comprising DNA, RNA, or protein, obtained from biopsies, blood, for example.

Provided with the information that the defect causing HNPCC and sporadic RER⁺ tumors is in a DNA mismatch repair enzyme, one can perform assays on test compounds and compositions to determine if they will remedy the defect. Such therapeutic compounds could bind to missense *hMSH* mutant proteins to restore the proteins to the normal, active conformation. Alternatively such therapeutic compounds could stimulate the expression of alternate pathways for mismatch repair. Screening for such therapeutic compounds could be performed by contacting test compounds with cells, either normal or those with an *hMSH2* mutation found in a tumor. The ability of the cells which were contacted with the test compounds is compared with the ability of the same cells which were not contacted for mismatch repair activity. Such activity can be tested as is known in the art. See, for example, Levinson and Gutman, 1987, and Strand et al., 1993. Observation of changes in microsatellite DNA in cells is one way of assessing mismatch repair activity. Another approach is to assay. DNA mismatch repair *in vitro* in nuclear extracts. See Holmes, 1990; Thomas, 1991; and Fang, 1993.

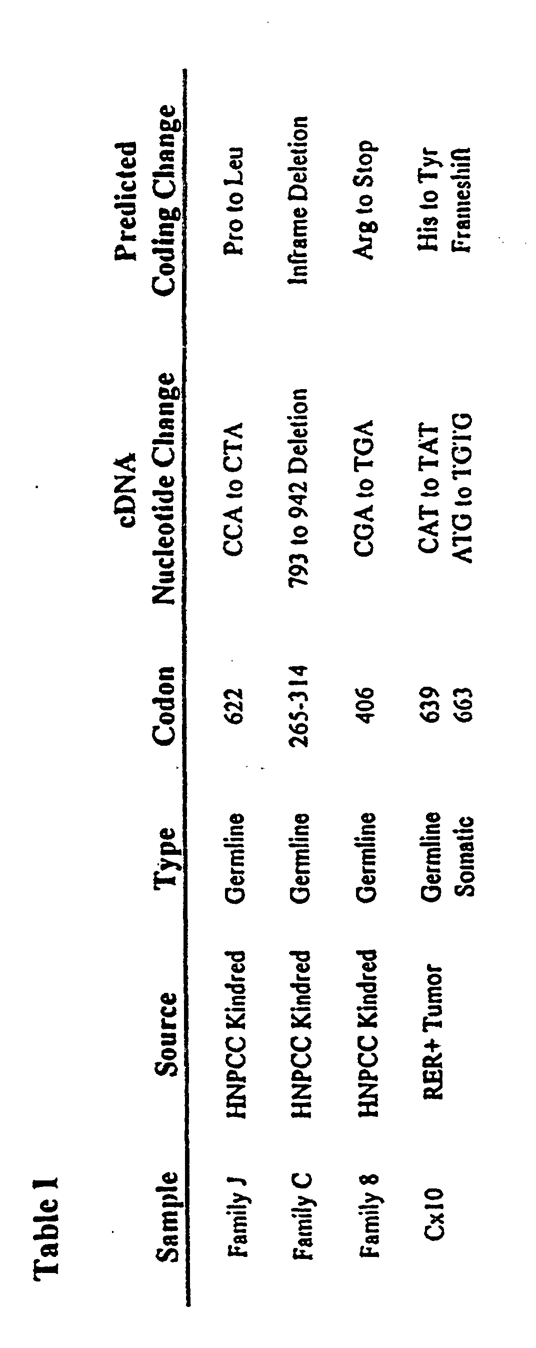

Provided with the cDNA sequence and the amino acid of *hMSH2* protein, one of ordinary skill in the art can readily produce *hMSH2* protein, isolated and purified from other human proteins. For example, recombinant cells or organisms can be used to produce the protein in bacteria, yeast, or other convenient cell system. The isolated and purified protein can be used in screening for new therapeutic agents, for example, in *in vitro* assays of DNA mismatch repair. The protein can also be used to raise antibodies against *hMSH2.* Therapeutic administration of the protein is also contemplated.

Transgenic animals are also contemplated by the present invention. These animals would have inserted in their germline *hMSH2* alleles which are associated with HNPCC or sporadic tumors. Such animals could provide model systems for testing drugs and other therapeutic agents to prevent or retard the development of tumors. Also contemplated are genetically engineered animals which contain one or more mutations in their own *MSH2* genes. The mutations will be engineered to correspond to mutations found in *hMSH2* alleles which are found in HNPCC-affected individuals or in other human RER⁺ tumors. Animals with both native *MSH2* alleles inactivated and containing a human wild-type or mutant *hMSH2* allele are particularly desirable.

### Examples

### Example 1

### Somatic Cell Hybrids

A panel of human-hamster, human-mouse, and human-rat hybrid cell lines was developed to facilitate HNPCC mapping. Hybrids containing only portions of chromosome 2 were obtained by microcell-mediated chromosome transfer or by standard cell fusions following X-irradiation of the chromosome 2 donor. Additionally, two hybrids were used which contained a (X;2)(q28;p21) translocation derived from human fibroblasts. In previous studies, the HNPCC locus was mapped to the 25 cM region surrounding marker 123 and bordered by markers 119 and 136 (Peltomaki et al., 1993a). Thirty-eight hybrids were screened with these three chromosome 2p markers. Eight of the hybrids proved useful for mapping the relevant portion of chromosome 2p. For example, hybrids L1 and L2 contained the distal half of the region, including marker 123, while hybrid y3 contained the half proximal to marker 123 (Figure 1).

### Methods

Methods for the derivation of microcell-mediated chromosome 2 hybrids have been described previously (Chen et aL, 1992; Spurr et al., 1993). Some hybrids were generated following fusion of X-irradiated donor cells containing human chromosome 2 to CHO cells (Chen et al., 1994). Mouse hybrids were derived by fusing HPRT deficient L cells (A9) with human fibroblasts (GM7503) containing a t(X;2)(q28,p21) translocation and selecting in media containing HAT.

### Example 2

### Polymorphic Markers

To map more finely the HNPCC locus, additional polymorphic markers were obtained in three ways. First, a genomic clone containing 85 kb surrounding the 123 marker was used for fluorescence *in situ* hybridization (FISH) to localize it to chromosomal band 2p16.3 (Figure 2A,B). The 2p16 band region was then microdissected, and the sequences within this band were amplified using the polymerase chain reaction and subcloned into plasmid vectors (see Experimental Procedures). The accuracy of the microdissection was confirmed using dual-color FISH by simultaneously hybridizing to microdissected material and a genomic clone containing marker 123 (Figure 2C,D). The subclones were screened by hybridization to a (CA)₁₅ probe, and hybridizing clones identified and sequenced. These sequences were then used to design oligonucleotide primers for PCR analysis of genomic DNA. Nineteen (CA)ₙ repeat markers were identified in this way. Of these, four were highly polymorphic and mapped to the region between markers 119 and 136, as assessed by the somatic cell hybrid panel exhibited in Figure 1. Second, eight additional (CA)ₙ markers, cloned randomly from human genomic DNA using a poly (CA) probe, were found to lie between markers 119 and 136 by linkage analysis in CEPH pedigrees. Five of these were particularly informative and were used in our subsequent studies. Finally, one additional marker was identified by screening subclones of a genomic P1 clone containing marker 123 with a (CA)₁₅ probe. Through these analyses, thirteen new polymorphic markers were identified in the 25 cM interval between markers 119 and 136, resulting in an average marker spacing of ~2 cM (Table II). These markers were mapped with respect to one another by linkage in CEPH and HNPCC pedigrees as well as by analysis of somatic cell hybrids. These two mapping techniques provided consistent and complementary information. For example, the relative positions of CA16 and CA18 could not be distinguished through linkage analysis but could be determined with the somatic cell hybrids L1, L2, and Y3. Conversely, the relative position of the ze3 and yh5 markers could not be determined through somatic cell hybrid mapping, but could be discerned by linkage analysis.

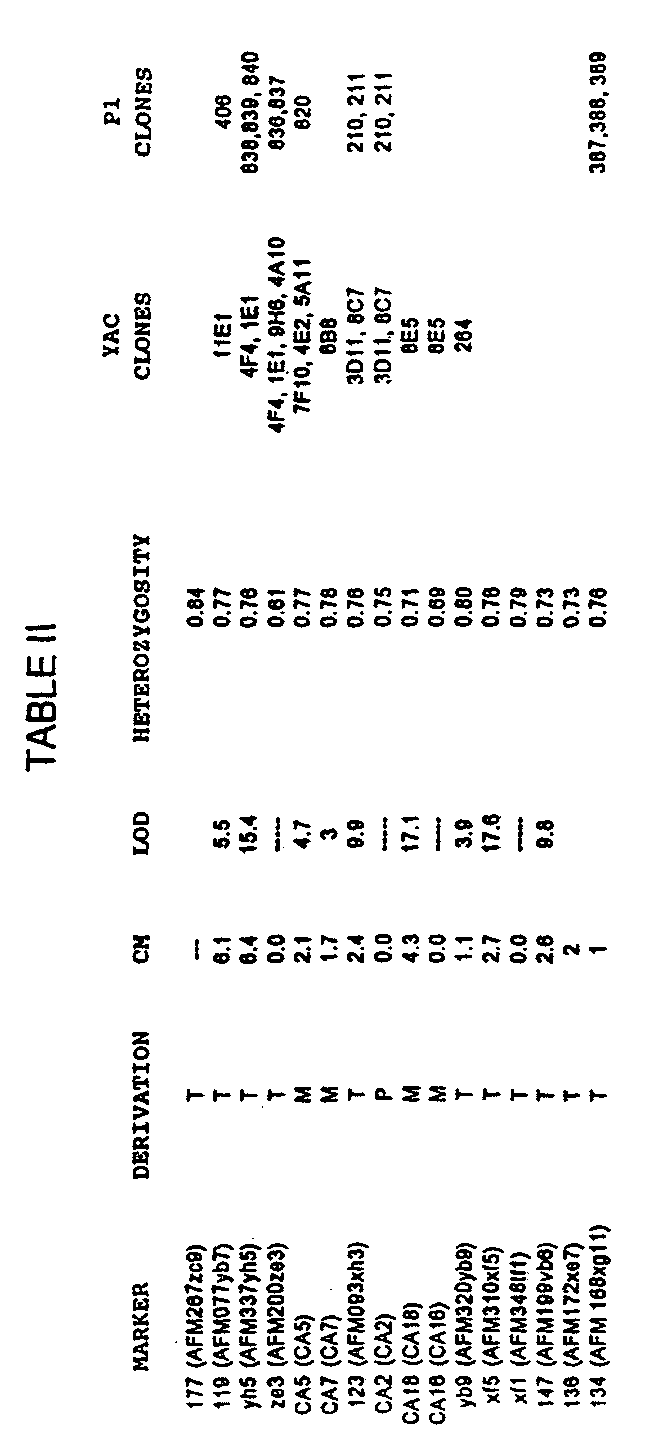

### Methods

All markers were obtained by screening human genomic libraries with radiolabelled (CA)ₙ probes (Weber and May, 1989). The "T" markers (see Table 1) were generated from a library made from total human genomic DNA, as described in Weissenbach et al., 1992. The "M" markers were made from libraries generated from microdissected chromosome 2p16, as described below. The CA2 marker was generated from a library made from P1 clone 210 digested to completion with Sau3 and cloned into the XhoI site of lambda YES (Elledge et al., 1991). The sequences of the clones obtained from these libraries were determined, and primers surrounding the CA repeats chosen. Only primers giving robust amplification and high heterozygosity were used for detailed analysis of HNPCC kindreds. All markers used in this study were shown to be derived from chromosome 2p by both linkage analysis in the CEPH pedigrees and evaluation in the somatic cell hybrid panel shown in Figure 1. The sequences of the primers and other details specific for each marker have been deposited with the Genome Data Base. Linkage analyses to obtain the map of the marker loci in CEPH families 1331, 1332, 1347, 1362, 1413, 1416, 884, and 102 (Weissenbach et al., 1992) were performed using the CLINK program of the LINKAGE program package (Lathrop et al., 1984) with the no sex difference option and 11-point computations. The odds for the best locus order supported by the data were evaluated against pairwise inversions of the loci.

### Example 3

### Genomic Clones

Many of the polymorphic markers shown in Table 1 were used to derive genomic clones containing 2p16 sequences. Genomic clones were obtained by PCR screening of human P1 and YAC libraries with these polymorphic markers, with ten additional sequence tagged sites (STS) derived from chromosome 2p16 microdissection, or with YAC junctions. Twenty-three P1 clones, each containing 85-95 kb, were obtained, as well as 35 YAC clones, containing 300 to 1800 kb. The YAC clones in some cases confirmed the linkage and somatic cell hybrid maps. For example, markers ze3 and yh5 were both found in YAC's 4F4 and 1E1 while CA16 and CA18 were both found in YAC 8E5, documenting their proximity. The highest density of genomic clones (28 YAC and 17 P1 clones) was obtained between markers yb9 and yh5 (Table 1), which became the region most likely to contain the HNPCC gene during the course of these studies. The region between yh5 and yb9 was predicted to contain ~ 9 Mb (assuming 1 Mb/per cM). Based on the sizes of the YAC clones, and taking into account their chimerism, we estimated that they contained over 70% of the sequences between yh5 and yb9.

### Methods

The markers described in Table 1 were used to screen YAC or P1 libraries by PCR. The CEPH A library was obtained from Research Genetics, Inc. and consisted of 21,000 YAC clones, arrayed in a format allowing facile screening and unambiguous identification of positive clones. The sizes of ten of the YAC clones containing markers were determined by transverse alternating pulse-field gel electrophoresis using a GeneLine II apparatus from Beckman and found to average 0.7 Mb (range 0.2-1.8 Mb). In some cases, inverse PCR was used to determine the YAC junctions (Joslyn et al., 1991), and the derived sequence used for "chromosome walking" with the YAC or P1 libraries. The junctions were also used to design primers to test whether the ends of the YAC clones could be localized to chromosome 2p16 (and therefore presumably be non-chimeric). Three of four YAC clones which were tested in this way had both ends within the expected region of chromosome 2, as judged by analysis with the somatic cell hybrid panel. The human genomic P1 library was also screened by PCR (Genome Systems, Inc.). P1 clones M1015 and M1016, containing the *hMSH2* gene, were used to determine intron-exon borders using sequencing primers from the exons and SequiTherm™ polymerase (Epicentre Technologies).

### Example 4

### Analysis of HNPCC Families

The markers described in Table 1 were then used to analyze six large HNPCC kindreds previously linked to chromosome 2p (Peltomaki et al., 1993a). Two hundred thirteen individuals, including 56 members affected with colorectal or endometrial cancer, were examined. Four of the kindreds were from the United States, one from Newfoundland and one from New Zealand. To increase the number of affected individuals that could be examined, we obtained formalin-fixed, paraffin-embedded sections of normal tissues from deceased individuals and purified DNA from them (Goelz et al., 1985a). A single allele of each of the thirteen markers was found to segregate with disease in each of the six families (i.e., the allele was found in over 50% of affected individuals). No allele of any marker was shared among the affected members of more than three kindreds.

Fourteen of the affected members contained only a subset of the expected alleles and therefore had undergone recombination between markers 119 and 136. Eleven of these individuals appeared to have simple, single recombination events. The most informative of these was in individual 148 from the J kindred and individual 44 from kindred 621 (Figure 3). Individual 621-44 apparently retained the disease linked allele at markers distal to CA5, while demonstrating multiple recombinants at more proximal loci, thus placing the CA5 marker at the proximal border of the HNPCC locus. Individual J-148 apparently retained the disease-linked allele at all markers proximal to yh5, while exhibiting recombinants at yh5 and 119, thus placing the distal border at yh5. Assuming that the same gene was involved in both the J and 621 kindreds, the HNPCC gene was predicted to reside between markers CA5 and yh5, an area spanning approximately 2 cM (Table 1).

The DNA of three affected individuals (C-202, 4-156, 4-92) appeared to have undergone two recombinations in the area. There was probably one recombinant per generation, and this could be demonstrated in C-202 by analysis of DNA from his parents; in the other cases, parental DNA was not available. All three individuals retained disease-linked alleles at CA5 and ze3 but not at more proximal and distal loci (Figure 3). Combined with the data from the patients with single recombinations, the double recombinants suggested that the HNPCC gene resided between CA5 and ze3, a distance spanning less than 2 cM.

To determine the physical distances separating CA5, ze3, and yh5, metaphase and interphase FISH analysis was carried out. Dual-color FISH with P1 clones containing these markers was performed with P1 clones 820 and 838 (containing the markers CA5 and yh5, respectively) labeled with biotin and detected with fluorescein-labelled avidin, and clone 836 (containing marker ze3) labelled with Spectrum-Orange (Meltzer et al., 1992). The hybridization signals of these markers appeared coincident on metaphase chromosomes, confirming that they resided within an interval of <1.0 Mb. When FISH was performed on interphase nuclei, the relative positions of the three markers could be determined and the distances between them estimated (Trask et al., 1989). The results confirmed that the orientation of the markers Was telomere-yh5-ze3-CA5-centromere (data not shown). Direct measurement of the distance between yh5 and ze3 was estimated at <0.3 Mb, consistent with the presence of both of these markers on YAC clones 4E4 and 1E1. Measurements of 48 interphase chromosomes provided an estimate of the distance between ze3 and CA5 at < 0.8 Mb, independently confirming the linkage data.

### Methods

G-banded metaphase chromosomes were microdissected with glass microneedles and amplified by PCR as previously described (Guan et al., 1993 Kao and Yu, 1991). For dual-color FISH, the PCR product was fluorochrome labelled (Spectrum-Orange, Imagenetics, Naperville, IL) or biotinylated in a secondary PCR reaction. P1 clones were labelled by nick-translation or by degenerate oligonucleotide primers (Guan et al., 1993). FISH was carried out as previously described (Guan et al., 1993) and visualized with a Zeiss Axiophot equipped with a dual-bandpass filter. For analysis of interphase FISH patterns, the distance between hybridization signals was measured in a minimum of 24 nuclei (Trask et al., 1989).

### Example 5

### Candidate Genes

On the basis of the mapping results described above, we could determine whether a given gene was a candidate for HNPCC by determining its position relative to the CA5-ze3 domain. The first gene considered was a human homolog of the Drosophila *SOS* gene (reviewed by Egan and Weinberg, 1993). This gene transmits signals from membrane bound receptors to the ras pathway in diverse eukaryotes. It was considered a candidate because another ras-interacting gene, *NF1,* causes a cancer predisposition syndrome (Viskochil et al., 1990; Wallace et al., 1990), and *SOS* has been localized to chromosome 2p16-21 by *in situ* hybridization (Webb et al., 1993). Using PCR to amplify *SOS* sequences from the hybrid panel, however, *SOS* was found to be distal to the CA5-yh5 domain (present in hybrid Z19 but not Z29).

We next examined the interferon-inducible RNA activated protein kinase gene *PKR.* This gene has been shown to have tumor suppressor ability (reviewed by Lengyel, 1993) and to map close to 2p16 (Hanash et al., 1993). We could not initially exclude *PKR* from the HNPCC domain, and therefore determined the sequence of its coding region in two individuals from HNPCC kindred C. Reverse transcriptase was used to generate cDNA from lymphoblastoid derived RNA of these two individuals, and PCR performed with primers specific for *PKR.* The *PKR* products were sequenced, and no deviations from the published sequence was identified within the coding region (Meurs et al., 1990). Subsequent studies showed that the *PKR* gene was distal to the yh5 marker, and thus could be excluded as a basis for HNPCC.

We then considered human homologs of the MutL and MutS mismatch repair genes previously shown to produce microsatellite instability in bacteria and yeast when disrupted (Levinson and Gutman, 1987; Strand et al., 1993). A human homolog of the yeast *MutL*-related gene *PMS1* (Kramer et al., 1989) does not appear to reside on chromosome 2p (M. Liskay, personal communication). To identify homologs of *MutS,* we used degenerate oligonucleotide primers to PCR-amplify cDNA from colon cancer cell lines. The same primers had been previously used to identify the yeast *MSH2* gene on the basis of its *MutS* homology (Reenan and Kolodner, 1992). Under non-stringent conditions of PCR, a fragment of the expected size was obtained and these fragments were cloned into plasmid vectors. Most of the clones contained ribosomal RNA genes, representing abundant transcripts with weak homology to the degenerate primers. A subset of the clones, however, contained sequences similar to that of the yeast *MSH2* gene, and one such clone, pNP-23, was evaluated further. The human gene from which this clone is derived is hereafter referred to as *hMSH2.*

The insert from clone pNP-23 was used as a probe in Southern blots of somatic cell hybrid DNA. This insert hybridized to one or two fragments in human genomic DNA digested with PstI or EcoRI, respectively, and these fragments were present in hybrid Z30, containing most of human chromosome 2p. Analysis of other hybrids showed that the fragment was present in hybrids Z11, Z29, L1 and L2, but not Z12, Y3 or Z19, thereby localizing the human *MSH2* (*hMSH2*) gene to a region bordered by markers CA18 and 119 (examples in Figure 4). The YAC clones listed in Table 1 were then analyzed, and EcoRI and PstI fragments of the expected size identified in YAC 5A11, derived from screening the YAC library with the CA5 marker (Figure 4).

To confirm the Southern blots, we designed non-degenerate primers on the basis of the sequence of pNP-23. Several sets of primers were tested so that genomic DNA could be used as a template for PCR; an intervening intron prevented the original primers from being used effectively with templates other than cDNA. PCR with these primers was perfectly consistent with the Southern blot results. The expected 101 bp fragment was present in hybrids Z4, Z11, Z29, L1, and L2, and in YAC 5A11, but not in other hybrids or YAC clones (not shown).

The localization of *hMSH2* sequences to YAC 5A11 demonstrated the proximity of these sequences to marker CA5. To determine the distance and relative orientation of *hMSH2* with respect to CA5, we performed interphase FISH analysis. P1 clones containing CA5, ze3 and *hMSH2* sequences (clones 820, 836, and M1015, respectively) were simultaneously hybridized to interphase nuclei following fluorescein and Spectrum Orange labelling (Meltzer et al., 1992). The results demonstrated that *MSH2* resides within the HNPCC locus defined by linkage analysis to lie between CA5 and ze3, and less than 0.3 Mb from marker CA5 (Figure 2E).

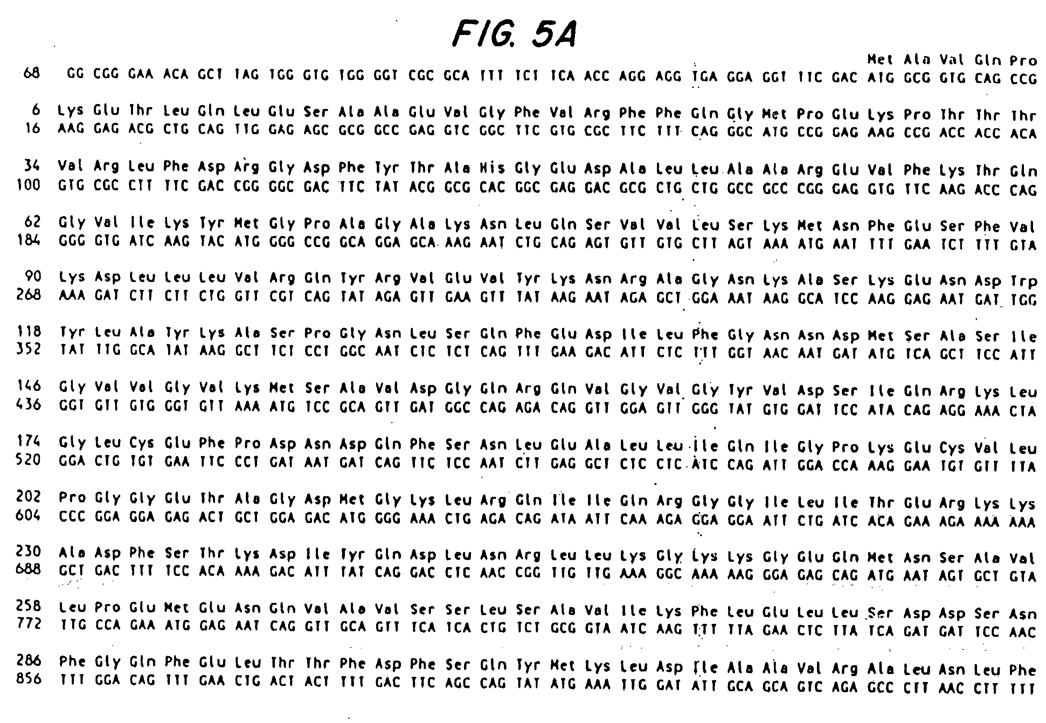

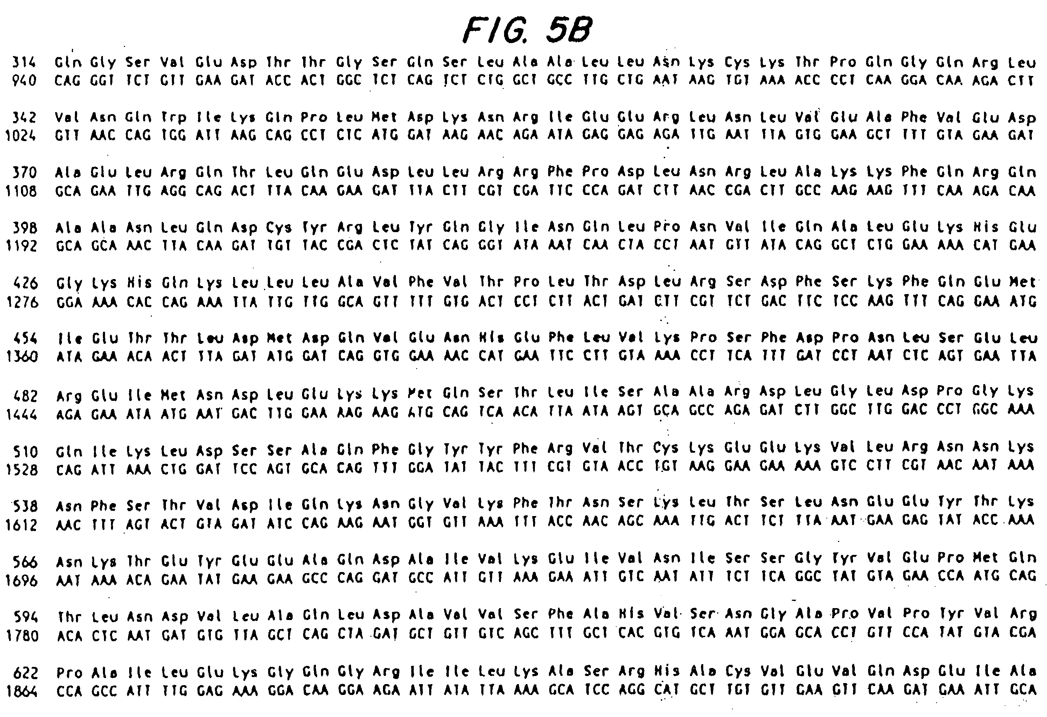

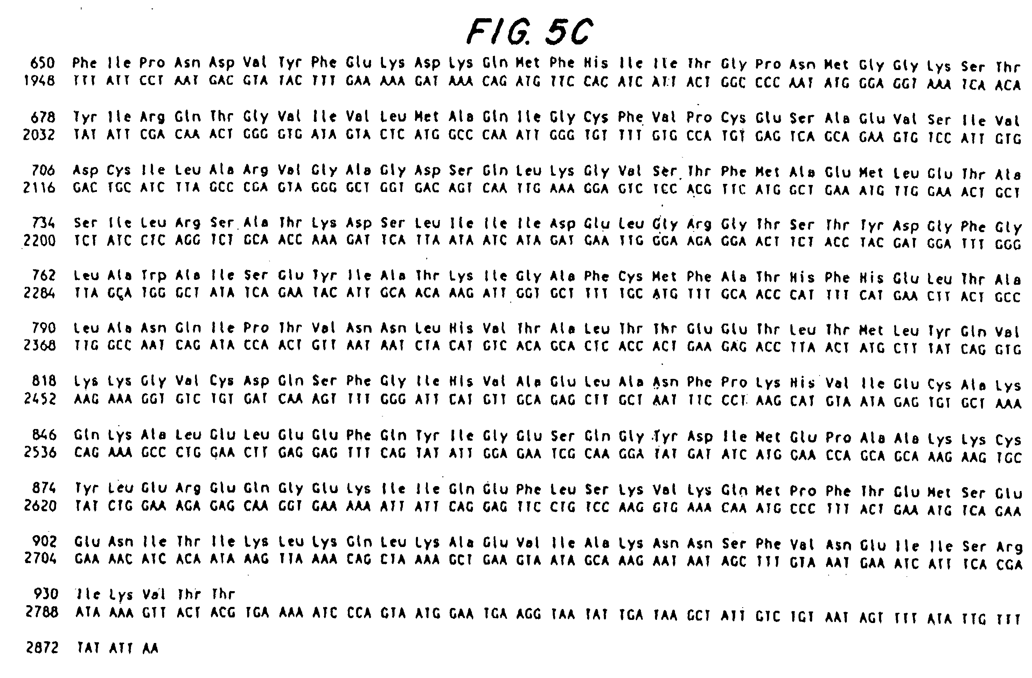

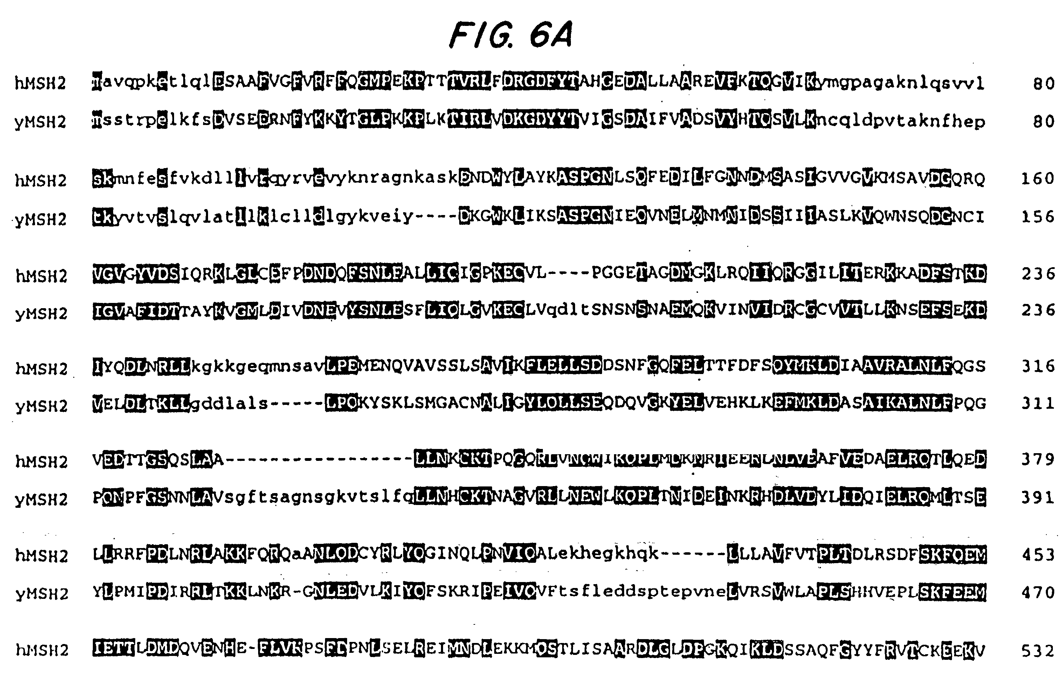

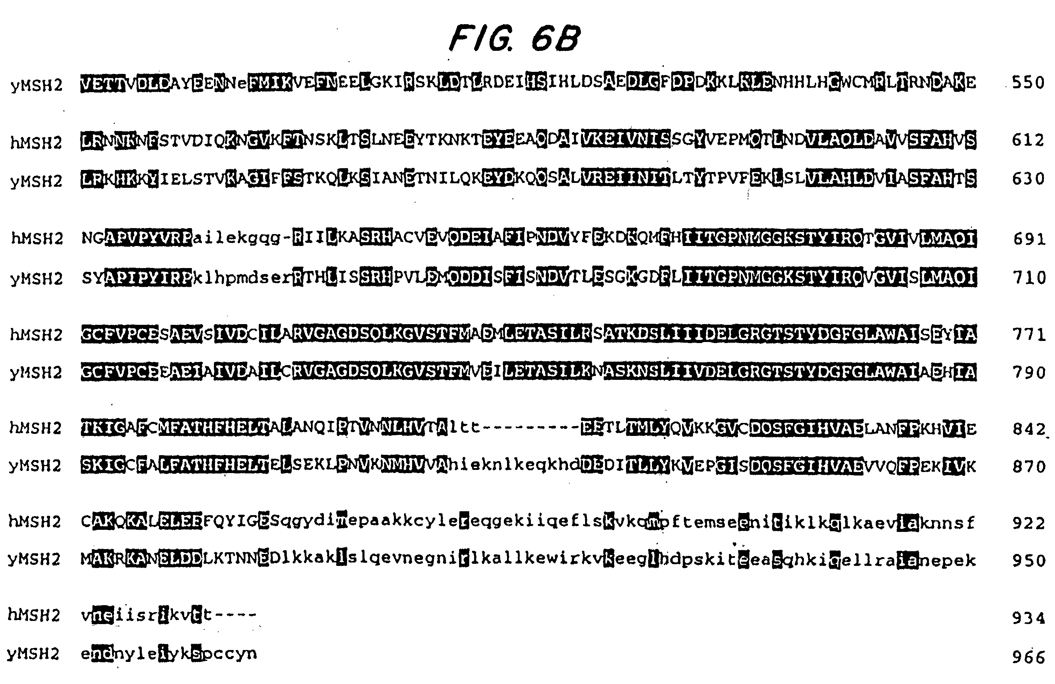

cDNA libraries generated from human colon cancer cells or from human fetal brain tissues were then screened with the insert of pNP-23 to obtain additional sequences from this gene. Seventy-five cDNA clones were initially identified and partially sequenced. PCR products representing the ends of the cDNA sequence contig were then used. as probes to re-screen the cDNA libraries. This cDNA "walk" was repeated again with the new contig ends. Altogether, 147 cDNA clones were identified. The composite sequence derived from these clones is shown in Figure 5. An open reading frame (ORF) began 69 nt downstream of the 5' end of the cDNA contig, and continued for 2802 bp. The methionine initiating this ORF was in a sequence context compatible with efficient translation (Kozak, 1986) and was preceded by in-frame termination codons. RNA from placenta and brain were used in a PCR-based procedure (RACE, Frohman et al., 1988) to independently determine the position of the 5' end of *hMSH2* transcripts. This analysis demonstrated that the 5' ends of all detectable transcripts were less than 100 bp upstream of the sequence shown in Figure 5, and were heterogeneous upstream of nt -69. The region of highest homology to the yeast *MSH2* gene is shown in Figure 6. This region encompassed the helix-turn-helix domain perhaps responsible for MutS binding to DNA (Reenan and Kolodner, 1992). The yeast and human *MSH2* proteins were 77% identical between codons 615 and 788. There were several other blocks of similar amino acids distributed throughout the length of these two proteins (966 and 934 amino acids in yeast and human, respectively).

### Methods

cDNA generated from the RNA of colorectal cancer cells with reverse transcriptase was used as template for PCR with the degenerate primers 5'-CTG GAT CCA C(G/A/T/C) G G(G/A/T/C)C C(G/A/T/C)A A(T/C)A TG-3' and 5'-CTG GAT CC(G/A) TA(G/A) TG(G/A/T/C) GT (G/A/T/C) (G/A)C(G/A) AA-3'. These two primers were used previously to identify the yeast MSH2 gene and were based on sequences conserved among related mammalian and bacterial genes (Reenan and Kolodner, 1992). The optimal PCR conditions for detecting the human *MSH2* gene consisted of 35 cycles at 95° for 30 seconds, 41° for 90 seconds, and 70° for 90 seconds, in the buffer described previously (Sidransky et al., 1991). PCR.products were cloned into T-tailed vectors as described (Holton and Graham, 1991) and sequenced with modified T7 polymerase (USB). The insert from one clone (pNP-23) containing human sequences homologous to the yeast *MSH2* gene was then used to screen cDNA libraries generated from RNA of SW480 colon cancer cells (Clontech) or of fetal brain (Stratagene). After two further rounds of screening, positive clones were converted into plasmids and sequenced using modified T7 polymerase (Kinzler et al., 1991). In some cases, the inserts were amplified using one *hMSH2*-specific primer and one vector-specific primer, and then sequenced with SequiTherm Polymerase (Epicentre Technologies). To determine the 5' end of *MSH2* transcripts, RACE was performed (Frohman et al., 1989) using brain and placenta cDNA (Clontech).

### Example 6

### Mutations of hMSH2

The physical mapping of *hMSH2* to the HNPCC locus was intriguing but could not prove that this gene was responsible for the disease. To obtain more compelling evidence, we determined whether germ line mutations of *hMSH2* were present in the two HNPCC kindreds that originally established linkage to chromosome 2 (Peltomaki et al., 1993a). Intron-exon borders within the most conserved region of *hMSH2* (Figure 6) were determined by sequencing genomic PCR fragments containing adjacent exons. Genomic DNA samples from the lymphocytes of affected members of these two kindreds were then used as templates for PCR to determine the sequence of this domain. The DNA from individual J-42, afflicted with colon and endometrial cancer at ages 42 and 44, respectively, was found to contain one allele with a C to T transition at codon 622 (CCA to CTA), resulting in a substitution of leucine for proline (Figure 7, top). Twenty additional DNA samples from unrelated individuals all encoded proline at this position. Twenty one members of the J kindred were then analyzed by direct sequencing of PCR products. All eleven affected individuals contained one allele with a C to T transition in codon 622, while all ten unaffected members contained two normal alleles, thus documenting perfect segregation with disease. Importantly, this proline was at a highly conserved position, the identical residue being found in all known MutS related genes from prokaryotes and eukaryotes (Figure 6 and Reenan and Kolodner, 1992).

No mutations of the conserved region of *MSH2* were identified in kindred C, so we next examined other parts of the *hMSB2* transcript. RNA was purified from lymphoblastoid cells of patient C-202, a 27 year old male with colon cancer. Reverse transcriptase coupled PCR (RT-PCR) was used to generate four *hMSH2*-specific products encompassing codons 89 to 934 from this RNA (see Experimental Procedures). An abnormal, smaller RT-PCR product was identified with one of the primer pairs used. Mapping and sequencing studies using various *MSH2* primers showed that the abnormal product was the result of a presumptive splicing defect which removed codons 265 to 314 from the *hMSH2* transcript. The abnormal transcript was found to segregate with disease in the C kindred, and was not found in twenty unrelated individuals.

We next wished to determine whether *hMSH2* was altered in one of the more recently linked families (R.P. and M.N-L., unpublished data) , and chose kindred 8 for detailed analysis. DNA and RNA were obtained from lymphoblastoid cells of 8-143, a 42 year old male with colon cancer. The conserved region of *hMSH2* was amplified from genomic DNA using PCR and directly sequenced. A T to C substitution was noted in the polypyrimidine tract upstream of the exon beginning at codon 669 (at intron position -6). However, this substitution was also found in two of twenty unrelated, normal individuals, and was therefore a polymorphism unrelated to the disease, with an allele frequency of 0.05. Most of the *hMSH2* coding region was then amplified by RT-PCR, as described above, and no abnormal transcripts were detected. Sequencing of the PCR products, however, revealed a C to T transition at codon 406 (CGA to TGA) causing substitution of a termination codon for an arginine residue. RNA was available from the lymphocytes of a second affected member of kindred 8, and the same stop codon was identified. This alteration was not found in twenty other, unrelated individuals.

Finally, we wished to determine whether mutations of this gene occurred in RER⁺ tumors from patients without evident family histories of cancer. The conserved region of *MSH2* was studied in four colorectal tumor cell lines from such patients using genomic DNA as templates for PCR. One tumor (from patient Cx10) was found to contain two *hMSH2* alterations. The first was a C to T transition in codon 639 (CAT to TAT), resulting in a substitution of tyrosine for histidine. This change was not found in any of twenty samples from unrelated individuals, but was present in the DNA from normal colon of this patient, and was therefore likely to represent a germ line change (Figure 7, middle). Like the missense mutation in the J kindred, the Cx10 alteration was at a position perfectly conserved in all MutS homologs (Figure 6 and Reenan and Kolodner, 1992). The second alteration in the tumor from Cx10 was a substitution of a GT dinucleotide for an A in codon 663 (ATG to TGTG). The resultant one bp insertion was predicted to cause a frameshift, producing a termination codon 36 nt downstream. This mutation was demonstrated in both RNA and DNA purified from the Cx10 tumor, but was not present in the patient's normal colon, so represented a somatic mutation (Figure 7, bottom). The PCR products from Cx10 were cloned and sequenced, and the insertion mutation at codon 663 and the transition at codon 639 were shown to reside on different alleles.

### Methods

To detect mutations, PCR products were generated from cDNA and human genomic DNA templates, then sequenced directly using SequiTherm™. In some cases, the PCR products were cloned into T-tailed vectors for sequencing to confirm the direct sequencing data. The primers used to amplify the conserved region *of MSH2* from genomic DNA were 5'-CCA CAA TGG ACA CTT CTG C-3' and 5'-CAC CTG TTC CAT ATG TAC G-3', resulting in a 1.4 kb fragment containing *hMSH2* codons 614 to 705, and primers 5'-AAA ATG GGT TGC AAA CAT GC-3' and 5'-GTG ATA GTA CTC ATG GCC C-3', resulting in a 2.0 kb fragment containing *MSH2* cDNA codons 683 to 783. Primers for RT-PCR were 5'-AGA TCT TCT TCT GGT TCG TC-3' and 5'-GCC AAC AAT AAT TTC TGG TG-3' for codons 89 to 433, 5'-TGG ATA AGA ACA GAA TAG AGG-3' and 5'-CCA CAA TGG ACA CTT CTG C-3' for codons 350-705, 5'-CAC CTG TTC CAT ATG TAC G-3' and 5'-AAA ATG GGT TGC AAA CAT GC-3' for codons 614 to 783, and 5'-GTG ATA GTA CTC ATG GCC C-3' and 5'-GAC AAT AGC TTA TCA ATA TTA CC-3' for codons 683-949.

### Discussion

Three major conclusions can be drawn from the examples described here. First, physical mapping and linkage analysis localized the HNPCC locus on chromosome 2 to an 0.8 Mb segment bordered by markers CA5 and ze3. Second, a new human homolog of the yeast *MSH2* gene was identified, and this gene shown to lie in the same 0.8 Mb interval. Third, alterations of the *hMSH2* gene occurred in the germ line of patients with RER⁺ tumors, with or without classical HNPCC, and additional somatic alterations of this gene occurred in tumors (Summarized in Table I). The alterations were at highly conserved regions or significantly altered the expected gene product and thus represent mutations with important functional effects. These results indicate that mutations of *hMSH2* are responsible for HNPCC and the RER⁺ positive phenotype found in tumors.

These data have substantial implications for understanding the neoplastic disease observed in HNPCC. In particular, they suggest that the microsatellite alterations previously observed in tumors from these patients are not epiphenomena, but are intrinsically related to pathogenesis. Additionally, the mutations observed in yeast and bacteria with defective MutS-related genes are not confined to insertions and deletions at simple repeated sequences, though these sequences provide convenient tools for analysis (Modrich, 1991). Similarly, one would expect that many mutations, in addition to microsatellite insertions or deletions, would be found in HNPCC tumors. This could lead to the multiple, sequential mutations in oncogenes and tumor suppressor genes which have been shown to drive colorectal tumorigenesis (Fearon and Vogelstein, 1990). Thus, the molecular pathogenesis of HNPCC tumors is likely to be similar to that occurring in non-HNPCC cases, though accelerated by the increased rate of mutation associated with mismatch repair defects. Accordingly, colon tumors from HNPCC patients have been shown to contain mutations of APC, p53 and RAS at frequencies similar to those found in sporadic colorectal cancers (Aaltonen et al. , 1993).

Colorectal tumors from HNPCC patients are distinguished by their relatively normal cytogenetic composition (Kouri et al., 1990), and sporadic, RER⁺ tumors have been demonstrated to have substantially fewer chromosome losses than those occurring in RER cases (Thibodeau et al., 1993; Aaltonen et al., 1993). These data suggest that genetic heterogeneity is critical for colorectal cancer development, but can be generated in two different ways (Thibodeau et al., 1993). Most commonly, it develops through gross alterations resulting in aneuploidy, as suggested nearly eighty years ago (Boveri, 1914). In HNPCC-derived tumors and RER⁺ sporadic tumors, the diversity is presumably more subtle, consisting of multiple small sequence changes distributed throughout the genome. The latter mechanism of generating diversity may be less dangerous to the host, as HNPCC patients, as well as patients with RER⁺ sporadic tumors, appear to have a better prognosis than would be expected from histopathologic analysis of their tumors (Ionov et al.,; 1993; Thibodeau et al., 1993; Lothe et al., 1993; Lynch et al., 1993).

### References

Aaltonen, L.A., Peltomaki, P., Leach, F.S., Sistonen, P., Pylkkanen, L., Mecklin, J-P., Jarvinen, H., Powell, S.M., Jen, J., Hamilton, S.R., Petersen, G.M., Kinzler, K.W., Vogelstein, B., and de la Chapelle, A. (1993). Clues to the pathogenesis of familial colorectal cancer. Science 260, 812-816.
Baylin, S.B., Makos; M., Wu, J., C-Y, R-W., de Bustros, A., Vertino, P., and Nelkin, B.D. (1991). Abnormal patterns of DNA methylation in human neoplasia: Potential consequences for tumor progression. Cancer Cells 3, 383, 390.
Boveri, T. (1914). Zur Frage der Entstehung maligner tumoren. Gustave Fischer Verlag, Jena, Vol. 1.
Chen, D.J., Park, M.S., Campbell, E., Oshimura, M., Liu, P., Zhao, Y., White, B.F., and Siciliano, M.J. (1992). Assignment of a human DNA double-strand break repair gene (XRCC5) to chromosome 2. Genomics 13, 1088-1094.
Chen, D.J., Marrone, B.., Nguyen, T., Stackhouse, M., Zhao, Y., and Sicilano, J.J., (1994). Regional assignment of a human radiation repair gene (XRCC5) to 2q35 by X-ray hybrid mapping. Genomics, in press.
Egan, S.E., and Weinberg, R.A. (1993). The pathway to signal achievement. Nature 365, 781-783.
Elledge, S.J., Mulligan, J.T., Ramer, S.W., Spottswood, M., and Davis R.W. (1991). Lambda YES: a multifunctional cDNA expression vector for the isolation of genes by complementation of yeast and *Escherichia coli* mutations. Proc. Natl. Acad. Sci. USA 88, 1731-1735.
Fang, W.-h. and P. Modrich (1993). Human strand-specific mismatch repair occurs by a bidirectional mechanism similar to that of the bacterial reaction. J. Biol. Chem. 268, 11838-11844.
Fearon, E.R., and Vogelstein, B. (1990). A genetic model for colorectal tumorigenesis. Cell 61, 759-767.
Frohman, M.A, Dush, M.K., and Martin, G.R. (1988). Rapid production of full-length cDNAs from rare transcripts: Amplification using a single gene-specific oligonucleotide primer. Proc. Natl. Acad. Sci. USA 85, 8998-9002.
Goelz, S.E., Hamilton, S.R., and Vogelstein, B. Purification of DNA from Formaldehyde Fixed and Paraffin Embedded Human Tissue. (1985a). Biochem. Biophys. Res. Commun. 130, 118-126.
Goelz, S.E., Vogelstein, B., Hamilton, S.R., and Feinberg, A.P. Hypomethylation of DNA from Benign and Malignant Human Colon Neoplasms. (1985b). Science 228, 187-190.
Guan, X.-Y., Trent, J.M., and Meltzer, P.S. (1993). Generation of band-specific painting probes from a single microdissected chromosome. Human Molecular Genetics 2, 1117-1121.
Han, H-J., Yanagisawa, A., Kato, Y., Park, J-G., and Nakamura, Y. (1993). Genetic instability in pancreatic cancer and poorly differentiated type of gastric cancer. Cancer Research 53, 5087-5089.
Hanash, S.M., Beretta, L., Barcroft, C.L., Sheldon, S., Glover, T.W., Ungar, D., and Sonenberg, N. (1993). Mapping of the gene for interferon-inducible dsRNA-dependent protein kinase to chromosome region 2p21-22: A site of rearrangements in myeloproliferative disorders. Genes, Chromosomes & Cancer 8, 34-37.
Holmes, J., S. Clark and P. Modrich (1990). Strand-specific mimsatch correction in nuclear extracts of human and *Drosophila melanogaster* cell lines. Proc. Natl. Acad. Sci. U.S.A. 87, 5837-5841.
Holton, T.A., and Graham, M.W. (1991). A simple and efficient method for direct cloning of PCR products using ddT-tailed vectors. Nucleic Acids Research 19, 1156.
Ionov, Y.M., Peinado, A., Malkhosyan, S., Shibata, D., and Perucho M. (1993). Ubiquitous somatic mutations in simple repeated sequences reveal a new mechanism for colonic carcinogenesis. Nature 363, 558-561.
Jass, J.R., Stewart, S.M. (1992). Evolution of hereditary non-polyposis colorectal cancer. Gut 33, 783-786.
Joslyn, G., Carlson, M., Thilveris, A., Albertsen, H., Gelbert, L., Samowitz, W., Groden, J., Stevens, J., Spirio, L., Robertson, M., Sargeant, L., Krapcho, K., Wolff, E., Burt, R., Hughes, J.P., Warrington, J., McPherson, J., Wasmuth, J., Le Paslier, D., Abderrahim, H., Cohen, D., Leppert, M., and White, R. (1991). Identification of deletion mutations and three new genes at the familial polyposis locus. Cell 66, 601-613.
Kao, F-T., and Yu, J-W. (1991). Chromosome microdissection and cloning in human genome and genetic disease analysis. Proc. Natl. Acad. Sci. USA 88, 1844-1848.
Kinzler, K.W., Nilbert, M.C., Su, L.-K., Vogelstein, B., Bryan, T.M., Levy, D.B., Smith, KJ., Preisinger, A.C., Hedge, P., McKechnie, D., Finniear, R., Markham, A., Groffen, J., Boguski, M.S., Altschul, S.F., Horii, A., Ando, H., Miyoshi, Y., Miki, Y., Nishisho, L, Nakamura, Y. Identification of FAP Locus Genes from Chromosome 5q21. (1991). Science 253, 661-665.
Kouri, M., Laasonen, A., Mecklin, J.P., Jarvinen, H., Franssila, K., Pyrhonen, S. (1990). Diploid predominance in hereditary nonpolyposis colorectal carcinoma evaluated by flow cytometry. Cancer 65, 1825-1829.
Kozak, M. (1986). Point mutations define a sequence flanking the AUG initiator codon that modulates translation by Eukaryotic Ribosomes. Cell 44, 283-292.
Kramer, W., Kramer, B., Williamson, M.S., Fogel, S. (1989). Cloning and nucleotide sequence of DNA mismatch repair gene PMS1 from Saccharomyces cerevisiae: homology of PMS1 to procaryotic MutL and HexB. Journal of Bacteriology 171, 5339-5346.
Knudson, A.G. (1993). All in the (cancer) family. Nature Genetics 5, 103-104.
Kunkel, T.A. (1993). Slippery DNA and diseases. Nature 365, 207-208.
Lathrop, G.M., Lalouel, J.M., Julier, C., and Ott, J. (1984). Strategies for multilocus linkage analysis in humans. Proc. Natl. Acad. Sci. USA 81, 3443-3446.
Lengyel, P. (1993). Tumor-suppressor genes: News about the interferon connection. Proc. Natl. Acad. Sci. USA 90, 5893-5895.
Levinson, G., and Gutman, G.A. (1987). High frequencies of short frameshifts in poly-CA/TG tandem repeats borne by bacteriophage M13 in *Escherichia coli* K-12. Nucleic Acids Research 15, 5323-5338.
Lindblom, A., Tannergard, P., Werelius, B., and Nordenskjold, M. (1993). Genetic mapping of a second locus predisposing to hereditary non-polyposis colon cancer. Nature Genetics 5, 279-282.
Lothe, R.A., Peltomaki, P., Meling, G-I, Aaltonen, L.A., Nystrom-Lahti, M., Pylkkanen, L., Heimdal, K., Andersen, T.I., Moller, P., Rognum, T.O., Fossa, S.D., Haldorsen, T., Langmark, F., Bragger, A., de la Chapelle, A., and Barresea, A-L. (1993). Genomic instability in colorectal caner: Relationship to clinicopathological variables and family history. Cancer Research, in press.
Lynch, H.T., Omaha, M.W., Shaw, M.D. (1966). Hereditary factors in cancer. Arch Intern Med 117, 206-212.
Lynch, H.T., Smyrk, T.C., Watson, P., Lanspa, S.J., Lynch, J.F., Lynch, P.M., Cavalieri, R.J., and Boland, C.R. (1993). Genetics, natural history, tumor spectrum, and pathology of hereditary nonpolyposis colorectal cancer: An updated review. Gastroenterology 104, 1535-1549.
Meltzer, P.S., Guan, X-Y., Burgess, A., and Trent, J.M. (1992). Rapid generation of region specific probes by chromosome microdissection and their application. Nature Genetics 1, 24-28.
Meurs, E., Chong, K., Galabru, J., Thomas, N.S.B., Kerr, I.M., Williams, B.R.G., and Hovanessian, A.G. (1990). Molecular cloning and characterization of the human double-stranded RNA-activated protein kinase induced by interferon. Cell 62, 379-390.
Modrich, P. (1991). Mechanisms and biological effects of mismatch repair. Ann. Rev. Genet. 25, 229-253.
Peinado, M.A., Malkhosyan, S., Velazquez, A., and Perucho, M. (1992). Isolation and characterization of allelic losses and gains in colorectal tumors by arbitrarily primed polymerase chain reaction. Proc. Natl. Acad. Sci. USA 89, 10065-10069.
Peltomaki, P., Aaltonen, L.A., Sistonen, P., Pylkkanen, L., Mecklin, J-P., Jarvinen, H., Green, J.S., Jass, J.R., Weber, J.L., Leach, F.S., Petersen, G.M., Hamilton, S.R., de la Chapelle, A., and Vogelstein, B. (1993a). Genetic mapping of a locus predisposing to human colorectal cancer. Science 260, 810-812.
Pukkila, P.J., Petseon, J., Herman, G., Modrich, P., and Meselson, M. (1983). Effects of high levels of DNA adenine methylation on methyl-directed mismatch repair in *Escherichia coli.* Genetics 104, 571-582.
Reenan, R.A., Kolodner, R.D. (1992). Isolation and characterization of two Saccharomyces cerevisiae genes encoding homologs of the bacterial HexA and MutS mismatch repair proteins. Genetics 132, 963-973.
Risinger, J.I., Berchuck, A., Kohler, M.F., Watson, P., Lynch, H.T., and Boyd, J. (1993). Genetic instability of microsatellites in endometrial carcinoma. Cancer Research 53, 5100-5103.
Sidransky, D., von Eschenbach, A., Tsai, Y.C., Jones, P., Summerhayes, I., Marshall, F., Paul, M., Green, P., Hamilton, S.R., Frost, P., Vogelstein, B. (1991). Identification of p53 Gene Mutations in Bladder Cancers and Urine Samples. Science 252, 706-709.
Spurr, N.K., Cox, S., Naylor, S. (1993). Report and abstracts of the Second International Workshop on human chromosome 2 mapping. Cytogenetics & Cell Genetics 64, 69-92.
Stanbridge, E.J. (1990). Human tumor suppressor genes. Ann. Rev. Genes 24, 615-657.
Strand, M., Prolla, T.A., Liskay, R.M., and Petes, T.D. (1993). Destabilization of tracts of simple repetitive DNA in yeast by mutations affecting DNA mismatch repair. Nature 365, 274-276.
Thibodeau, S.N., Bren, G., and Schaid, D. (1993). Microsatellite instability in cancer of the proximal colon. Science 260, 816-819.
Thomas, D.C., J.D. Roberts and T.A. Kunkel (1991). Heteroduplex repair in extracts of human HeIa cells. J.Biol. Chem. 266, 3744-3751.
Trask, B., Pinkel, D., and van den Engh, G. (1989). The proximity of DNA sequences in interphase cell nuclei is correlated to genomic distance and permits ordering of cosmids spanning 250 kilbase pairs. Genomics 5, 710-717.
Viskochil, D., Buchberg, A.M., Xu, G., Cawthon, R., Stevens, J., Wolff, R.K., Culver, M., Carey, J.C., Copeland, N.G., Jenkins, N.A., White, R., and O'Connell, P. (1990). Deletions and a translocation interrupt a cloned gene at the neurofibromatosis type 1 locus. Cell 62, 187-192.
Vogelstein, B., Fearon, E.R., Hamilton, S.R., Preisinger, A.C., Willard, H.F., Michelson, A.M., Riggs, A.D., and Orkin, S.H. (1987). Clonal Analysis Using Recombinant DNA Probes from the X Chromosome. Cancer Research 47, 4806-4813.
Wallace, M.R., Marchuk, D.A., Andersen, L.B., Letcher, R., Odeh, H.M., Saulino, A.M., Fountain, J.W., Brereton, A., Nicholson, J., Mitchell, A.L., Brownstein, B.H., Collins, F.S. (1990). Type 1 neurofibromatosis gene: Identification of a large transcript disrupted in three NF1 patients. Science 249, 181-186.
Warthin, A.S. (1913). Heredity with reference to carcinoma: As shown by the study of the cases examined in the pathological laboratory of the University of Michigan, 1895-1913. Arch. Int. Med 12, 546-555.
Watson, P., and Lynch, H.T. (1993). Extracolonic cancer in hereditary nonpolyposis colorectal cancer. Cancer 71, 677-685.
Webb, G.C., Jenkins, N.A., Largaespada, D.A., Copeland, N.G., Femandez, C.S., and Bowtell, D.D.L. (1993). Mammalian homologues of the Drosophila Son of sevenless gene map to murine chromosomes 17 and 12 and to human chromosomes 2 and 14, respectively. Genomics 18, 14-19.
Weber, J.L., May P.E. (1989). Abundant class of human DNA polymorphisms which can be typed using the polymerase chain reaction. American Journal of Human Genetics 44, 338-396.
Weissenbach, J., Gyapay, G., Dib, C., Vignal A., Morissette, J., Millasseau, P., Vaysseix, G., and Lathrop, M. (1992). A second-generation linkage map of the human genome. Nature 359, 794-801.

## Claims

1. An isolated and purified DNA molecule having an *hMSH2* sequence of at least about 20 nucleotides *of hMSH2* shown in SEQ ID NO: 1, wherein the *hMSH2* sequence does not consist of the sequence: or

2. The DNA molecule of claim 1 which is cDNA.

3. The DNA molecule of claim 1 or 2 which is labelled.

4. The DNA molecule of claim 1, 2 or 3 which is operably linked to a promoter sequence.

5. The DNA molecule of claim 4 which upon transcription produces an RNA molecule having the sequence of native *hMSH2* mRNA.

6. The isolated and purified DNA molecule of claim 1 which encodes the complete *hMSH2* protein as shown in SEQ ID NO: 2.

7. The isolated and purified DNA molecule of claim 1 which comprises the sequence shown in SEQ ID NO: 1.

8. An isolated and purified molecule having a sequence of at least about 20 nucleotides of an *hMSH2* allele found in a tumour wherein said DNA molecule contains a mutation relative to *hMSH2* shown in SEQ ID NO: 1, wherein the mutation is selected from the group consisting of a nonconservative amino acid substitution, a deletion, a premature termination and a frameshift mutation.

9. The isolated and purified DNA molecule of claim 8 wherein the tumour is an RER⁺ tumour.

10. The isolated and purified DNA molecule of claim 8 wherein said mutation is a C to T transition at nucleotide 1934 as shown in SEQ ID NO: 1 (codon 622 as shown in Figure 5B) which changes the encoded amino acid from a proline to a leucine.

11. The isolated and purified DNA molecule of claim 8 wherein said mutation is a deletion of nucleotides 793 to 942 as shown in SEQ ID NO: 1.

12. The isolated and purified DNA molecule of claim 8 wherein said mutation is a C to T transition at nucleotide 1285 as shown in SEQ ID NO: 1 (codon 406 as shown in Figure 5A) which changes an arginine to a stop codon.

13. The isolated and purified DNA molecule of claim 8 wherein said mutation is a C to T transition at nucleotide 1984 as shown in SEQ ID NO: 1 (codon 639 as shown in Figure 5B) which changes a histidine to a tyrosine.

14. The isolated and purified DNA molecule of claim 8 wherein said mutation is a frameshift at nucleotide 2056 as shown in SEQ ID NO: 1 (codon 663 as shown in Figure 5B) which changes an ATG codon to TGTG.

15. A method of determining a predisposition to cancer comprising:
testing a body sample of a *human in vitro* to ascertain the presence of a mutation in *hMSH2* as shown in SEQ ID NO: 1 which affects *hMSH2* expression or *hMSH2* protein function, the presence of such a mutation indicating a predisposition to cancer.

16. The method of claim 15 wherein the sample is DNA.

17. The method of claim 15 wherein the sample is RNA.

18. The method of claim 15 wherein the sample is protein.

19. The method of claim 15 wherein the sample is isolated from prenatal or embryonic cells.

20. A method of screening to identify therapeutic agents which can prevent or ameliorate tumours, comprising:
contacting a test compound with an isolated cell, wherein the cell contains *hMSH2* mutation found in a tumour;
determining the ability of the cell to perform DNA mismatch repair, wherein a test compound which increases the ability of said cell to perform DNA mismatch repair is a potential therapeutic agent.

21. A method of screening to identify therapeutic agents which can prevent or ameliorate tumours, comprising:
contacting a test compound with an isolated and purified *hMSH2* protein;
determining the ability of the *hMSH2* protein to perform DNA mismatch repair, wherein a test compound which increases the ability of said protein to perform DNA mismatch repair is a potential therapeutic agent.

22. The method of claim 21 wherein the *hMSH2* protein contains a mutation found in a tumour.

23. An isolated and purified protein which has the sequence shown in SEQ ID NO: 2.

24. A non-human transgenic animal wherein the transgene is an allele of an *hMSH2* gene wherein the nucleotide sequence of the wild-type *hMSH2* gene is shown in SEQ ID NO: 1.

25. The non-human transgenic animal of claim 24 wherein the allele is a mutant allele of *hMSH2* found in a patient having hereditary non-polyposis colorectal cancer or in an RER⁺ tumour.

26. A non-human transgenic animal which contains no wild-type native *MSH2* allele.

27. The transgenic animal of claim 24 wherein said animal contains a mutated native MSH2 allele.

28. A composition for treating a person having an RER⁺tumour to prevent accumulation of somatic mutations leading to resistance to an anti-cancer therapeutic agent, the composition consisting of:
(a) a DNA molecule which has a sequence of at least about 20 nucleotides of *hMSH2* as shown in SEQ ID NO: 1, wherein said DNA molecule is sufficient to remedy a mutation in an *hMSH2* allele of the person; and
(b) an anti-neoplastic therapeutic agent.

29. The composition of claim 28 wherein the anti-neoplastic therapeutic agent is selected from the group consisting of: a hormone, radiation, a cytotoxic drug, a cytotoxin, and an antibody.

## Patentansprüche

1. Ein isoliertes und aufgereinigtes DNA-Molekül mit einer *hMSH2*-Sequenz von wenigstens 20 Nukleotiden von *hMSH2,* gezeigt in SEQ ID NO: 1, wobei die *hMSH*-Sequenz nicht aus der Sequenz: oder besteht.

2. Das DNA-Molekül nach Anspruch 1, welches cDNA ist.

3. Das DNA-Molekül nach Anspruch 1 oder 2, welches markiert ist.

4. Das DNA-Molekül nach Anspruch 1, 2 oder 3, welches operabel mit einer Promotorsequenz verbunden ist.

5. Das DNA-Molekül nach Anspruch 4, welches nach der Transkription ein RNA-Molekül produziert, das die Sequenz von nativer *hMSH2*-mRNA hat.

6. Das isolierte und aufgereinigte DNA-Molekül nach Anspruch 1, welches für das vollständige *hMSH2*-Protein, wie gezeigt in SEQ ID NO: 2, codiert.

7. Das isolierte und aufgereinigte DNA-Molekül nach Anspruch 1, welches die Sequenz umfaßt, die in SEQ ID NO: 1 gezeigt ist.

8. Ein isoliertes und aufgereinigtes Molekül mit einer Sequenz von wenigstens 20 Nukleotiden von einem *hMSH2*-Allel, wie es in einem Tumor gefunden wird, wobei das DNA-Molekül eine Mutation relativ zu *hMSH2,* das in SEQ ID NO: 1 gezeigt ist, enthält, wobei die Mutation ausgewählt ist aus der Gruppe, bestehend aus einer Substitution mit einer nicht-konservativen Aminosäure, einer Deletion, einer zu frühen Terminierung und einer Frameshift-Mutation.

9. Das isolierte und aufgereinigte DNA-Molekül nach Anspruch 8, wobei der Tumor ein RER⁺-Tumor ist.

10. Das isolierte und aufgereinigte DNA-Molekül nach Anspruch 8, wobei die Mutation ein C nach T-Übergang bei Nukleotid 1934, wie gezeigt in SEQ ID NO: 1 (Codon 622, wie gezeigt in Figur 5B), ist, welches die codierte Aminosäure von einem Prolin in ein Leucin verändert.

11. Das isolierte und aufgereinigte DNA-Molekül nach Anspruch 8, wobei die Mutation eine Deletion der Nukleotide 793 bis 942 ist, wie gezeigt in SEQ ID NO: 1.

12. Das isolierte und aufgereinigte DNA-Molekül nach Anspruch 8, wobei die Mutation ein C nach T-Übergang bei Nukleotid 1285, wie gezeigt in SEQ ID NO: 1 (Codon 406, wie gezeigt in Figur 5A), ist, welche ein Arginin in ein Stopcodon verwandelt.

13. Das isolierte und aufgereinigte DNA-Molekül nach Anspruch 8, wobei die Mutation ein C nach T-Übergang bei Nukleotid 1984, wie gezeigt in SEQ ID NO: 1 (Codon 639, wie gezeigt in Figur 5B), ist, welches ein Histidin in ein Tyrosin verwandelt.

14. Das isolierte und aufgereinigte DNA-Molekül nach Anspruch 8, wobei die Mutation ein Frameshift bei Nukleotid 2056, wie gezeigt in SEQ ID NO: 1 (Codon 663, wie gezeigt in Figur 5B), ist, welches ein ATG-Codon in TGTG verwandelt.

15. Ein Verfahren zum Bestimmen einer Veranlagung für Krebs, umfassend:
Testen einer Körperprobe eines Menschen in vitro, um die Gegenwart einer Mutation in *hMSH2,* wie gezeigt in SEQ ID NO: 1, festzustellen, welche die *hMSH2*-Expression oder die *hMSH2*-Proteinfunktion beeinflußt, wobei die Gegenwart solch einer Mutation die Veranlagung für Krebs anzeigt.

16. Das Verfahren nach Anspruch 15, wobei die Probe DNA ist.

17. Das Verfahren nach Anspruch 15, wobei die Probe RNA ist.

18. Das Verfahren nach Anspruch 15, wobei die Probe Protein ist.

19. Das Verfahren nach Anspruch 15, wobei die Probe aus vorgeburtlichen oder aus embryonalen Zellen isoliert wird.

20. Ein Verfahren zum Screenen, um therapeutische Mittel zu identifizieren, welche Tumoren vorbeugen können oder sie abschwächen können, umfassend:
In-Kontakt-Bringen einer Testverbindung mit einer isolierten Zelle, wobei die Zelle eine *hMSH2*-Mutation, die in einem Tumor gefunden wird, enthält;
Bestimmen der Fähigkeit der Zelle, eine DNA-Mismatch-Reparatur auszuführen, wobei eine Testverbindung, welche die Fähigkeit der Zelle erhöht, eine DNA-Mismatch-Reparatur auszuführen, ein potentielles therapeutisches Mittel ist.

21. Ein Verfahren zum Screenen, um therapeutische Mittel zu identifizieren, welche Tumoren vorbeugen können oder sie abschwächen können, umfassend:
In-Kontakt-Bringen einer Testverbindung mit einem isolierten und aufgereinigten *hMSH2-*Protein;
Bestimmen der Fähigkeit des *hMSH2*-Proteins, eine DNA-Mismatch-Reparatur auszuführen, wobei eine testverbindung, die die Fähigkeit des Proteins erhöht, eine DNA-Mismatch-Reparatur auszuführen und ein potentielles therapeutisches Mittel ist.

22. Das Verfahren nach Anspruch 21, wobei das *hMSH2*-Protein eine Mutation enthält, die in einem Tumor gefunden wird.

23. Ein isoliertes und aufgereinigtes Protein, welches die Sequenz hat, die in SEQ ID NO: 2 gezeigt ist.

24. Ein nicht-humanes transgenes Tier, wobei das Transgen ein Allel eines hMSH2-Gens ist, wobei die Nukleotidsequenz des Wildtyp-*hMSH2*-Gens in SEQ ID NO: 1 gezeigt ist.

25. Das nicht-humane transgene Tier nach Anspruch 24, wobei das Allel ein mutiertes Allel von *hMSH2* ist, das in einem Patienten, der ein erbliches non-Polyposis-kolorektales Karzinom hat, oder in einem RER⁺-Tumor gefunden wird.

26. Ein nicht-humanes transgenes Tier, welches kein natives *MSH2*-Allel des Wildtyps enthält.

27. Das transgene Tier nach Anspruch 24, wobei das Tier ein mutiertes natives *MSH2*-Allel enthält.

28. Eine Zusammensetzung zur Behandlung einer Person mit einem RER⁺-Tumor, um der Ansammlung von somatischen Mutationen vorzubeugen, die zur Resistenz gegenüber einem therapeutischen anti-Krebs-Mittel führt, wobei die Zusammensetzung aus folgendem besteht:
(a) einem DNA-Molekül, welches eine Sequenz von wenigstens etwa 20 Nukleotiden von *hMSH2,* wie gezeigt in SEQ ID NO: 1, enthält, wobei das DNA-Molekül ausreichend ist, um einer Mutation in einem *hMSH2*-Allel der Person abzuhelfen; und
(b) einem anti-neoplastischen therapeutischen Mittel.

29. Die Zusammensetzung nach Anspruch 28, wobei das antineoplastische therapeutische Mittel ausgewählt ist aus der Gruppe, bestehend aus: einem Hormon, Strahlung, einem zytotoxischen Medikament, einem Zytotoxin und einem Antikörper.

## Revendications

1. Molécule d'ADN isolée et purifiée ayant une séquence *hMSH2* d'au moins environ 20 nucléotides de *hMSH2* montrée dans SEQ ID NO: 1, dans laquelle la séquence *hMSH2* n'est pas constituée par la séquence : ou

2. Molécule d'ADN selon la revendication 1 qui est de l'ADNc.

3. Molécule d'ADN selon la revendication 1 ou 2 qui est marquée.

4. Molécule d'ADN selon la revendication 1, 2 ou 3 qui est liée de manière opérationnelle à une séquence promoteur.

5. Molécule d'ADN selon la revendication 4 qui, après transcription, produit une molécule d'ARN ayant la séquence de l'ARNm de *hMSH2* natif.

6. Molécule d'ADN isolée et purifiée selon la revendication 1 qui code pour la protéine *hMSH2* complète comme montrée dans SEQ ID NO : 2.

7. Molécule d'ADN isolée et purifiée selon la revendication 1 qui comprend la séquence montrée dans SEQ ID NO : 1.

8. Molécule isolée et purifiée ayant une séquence d'au moins environ 20 nucléotides d'un allèle de *hMSH2* trouvé dans une tumeur dans laquelle ladite molécule d'ADN contient une mutation par rapport à *hMSH2* montrée dans SEQ ID NO : 1, dans laquelle la mutation est sélectionnée dans le groupe constitué d'une substitution d'acide aminé non conservatrice, d'une délétion, d'une terminaison prématurée et d'une mutation de changement de phase.

9. Molécule d'ADN isolée et purifiée selon la revendication 8, dans laquelle la tumeur est une tumeur RER+.

10. Molécule d'ADN isolée et purifiée selon la revendication 8, dans laquelle ladite mutation est une transition de C en T au niveau du nucléotide 1934 comme montrée dans SEQ ID NO : 1 (codon 622 comme montré à la figure 5B), ce qui change l'acide aminé codé d'une proline en une leucine.

11. Molécule d'ADN isolée et purifiée selon la revendication 8, dans laquelle ladite mutation est une délétion des nucléotides 793 à 942 comme montré dans SEQ ID NO : 1.

12. Molécule d'ADN isolée et purifiée selon la revendication 8, dans laquelle ladite mutation est une transition de C en T au niveau du nucléotide 1285 comme montrée dans SEQ ID NO : 1 (codon 406 comme montré à la figure 5A), ce qui change une arginine en un codon de terminaison.

13. Molécule d'ADN isolée et purifiée selon la revendication 8, dans laquelle ladite mutation est une transition de C en T au niveau du nucléotide 1984 comme montrée dans SEQ ID NO: 1 (codon 639 comme montré à la figure 5B), ce qui change une histidine en une tyrosine.

14. Molécule d'ADN isolée et purifiée selon la revendication 8, dans laquelle ladite mutation est un changement de phase au niveau du nucléotide 2056 comme montré dans SEQ ID NO: 1 (codon 663 comme montré à la figure 5B), ce qui change un codon ATG en TGTG.

15. Procédé de détermination d'une prédisposition au cancer comprenant :
le test d'un échantillon organique d'un humain in vitro pour vérifier la présence d'une mutation dans hMSH2 comme montrée dans SEQ ID NO : 1 qui affecte l'expression de hMSH2 ou la fonction de la protéine hMSH2, la présence d'une telle mutation indiquant une prédisposition au cancer.

16. Procédé selon la revendication 15, dans lequel l'échantillon est de l'ADN.

17. Procédé selon la revendication 15, dans lequel l'échantillon est de l'ARN.

18. Procédé selon la revendication 15, dans lequel l'échantillon est une protéine.

19. Procédé selon la revendication 15, dans lequel l'échantillon est isolé de cellules prénatales ou embryonnaires.

20. Procédé de criblage pour identifier des agents thérapeutiques qui peuvent prévenir ou améliorer les tumeurs, comprenant :
la mise en contact d'un composé d'essai avec une cellule isolée, dans laquelle la cellule contient une mutation *hMSH2* trouvée dans une tumeur ;
la détermination de la capacité de la cellule à effectuer une réparation des mésappariements de l'ADN,
dans lequel un composé d'essai qui augmente la capacité de ladite cellule à effectuer la réparation des mésappariements de l'ADN est un agent thérapeutique potentiel.

21. Procédé de criblage pour identifier des agents thérapeutiques qui peuvent prévenir ou améliorer les tumeurs, comprenant :
la mise en contact d'un composé d'essai avec une protéine *hMSH2* isolée et purifiée ;
la détermination de la capacité de la protéine *hMSH2* à effectuer une réparation des mésappariements de l'ADN,
dans lequel un composé d'essai qui augmente la capacité de ladite protéine à effectuer la réparation des mésappariements de l'ADN est un agent thérapeutique potentiel.

22. Procédé selon la revendication 21, dans lequel la protéine *hMSH2* contient une mutation trouvée dans une tumeur.

23. Protéine isolée et purifiée qui a la séquence montrée dans SEQ ID NO : 2.

24. Animal transgénique non humain, dans lequel le transgène est un allèle d'un *gène hMSH2* dans lequel la séquence de nucléotides du gène *hMSH2* de type sauvage est montrée dans SEQ ID NO : 1.

25. Animal transgénique non humain selon la revendication 24, dans lequel l'allèle est un allèle mutant de *hMSH2* trouvé chez un patient ayant un cancer colo-rectal non polyposique héréditaire ou dans une tumeur RER+.

26. Animal transgénique non humain qui ne contient pas d'allèle *MSH2* natif de type sauvage.

27. Animal transgénique selon la revendication 24, dans lequel ledit animal contient un allèle MSH2 natif muté.

28. Composition pour le traitement d'une personne ayant une tumeur RER+ pour prévenir l'accumulation de mutations somatiques conduisant à une résistance à un agent thérapeutique anticancéreux, la composition comprenant :
(a) une molécule d'ADN qui a une séquence d'au moins environ 20 nucléotides de *hMSH2* comme montrée dans SEQ ID NO : 1, dans laquelle ladite molécule d'ADN est suffisante pour compenser une mutation dans un allèle *hMSH2* de la personne et
(b) un agent thérapeutique anti-néoplasique.

29. Composition selon la revendication 28, dans laquelle l'agent thérapeutique anti-néoplasique est sélectionné dans le groupe consistant en une hormone, un rayonnement, un médicament cytotoxique, une cytotoxine et un anticorps.
